# EUROPÄISCHE PATENTSCHRIFT

(11) **EP 2 430 065 B1**
(45) Veröffentlichungstag und Bekanntmachung des Hinweises auf die Patenterteilung: **19.03.2014**
(21) Anmeldenummer: 10715887.5
(22) Anmeldetag: 04.05.2010
(51) Int. Cl.: C08G 64/02, A01N 25/30

(54) **HYPERVERZWEIGTE POLYCARBONATE ZUR SOLUBILISIERUNG SCHWERLÖSLICHER WIRKSTOFFE**
HYPERBRANCHED POLYCARBONATES FOR SOLUBILIZING POORLY SOLUBLE ACTIVE SUBSTANCES
POLYCARBONATE HYPER-RAMIFIÉ POUR LA SOLUBILISATION DE SUBSTANCES ACTIVES DIFFICILEMENT SOLUBLES

(30) Priorität: 11.05.2009 EP 09159881
(43) Veröffentlichungstag der Anmeldung: 21.03.2012
(73) Patentinhaber: BASF SE, 67056 Ludwigshafen (DE)
(72) Erfinder: TÜRK, Holger, 68161 Mannheim (DE); HABERECHT, Monika, 67063 Ludwigshafen (DE); ISHAQUE, Michael, 68165 Mannheim (DE); YAMADA, Hiroe, 66111 Saarbrücken (DE); SCHÖNFELDER, Daniel, B-1000 Bruxelles (BE); BRUCHMANN, Bernd, 67251 Freinsheim (DE)
(86) Internationale Anmeldenummer: PCT/EP2010/056001
(87) Internationale Veröffentlichungsnummer: WO 2010/130599

(56) Entgegenhaltungen:
- WO-A1-2008/040786
- WO-A1-2009/021986
- US-A1- 2008 167 430
- US-A1- 2009 030 140
- US-A1- 2009 099 319

## Beschreibung

Gegenstand der vorliegenden Erfindung ist eine Zusammensetzung enthaltend ein Amphiphil und einen Wirkstoff, der in Wasser bei 20 °C zu höchstens 10 g/L löslich ist. Weitere Gegenstand ist ein Amphiphil enthaltend ein hyperverzweigtes Polycarbonat, das verknüpft ist mit mindestens einem linearen oder Kamm-förmigen Polymer, ein Verfahren zur Herstellung des Amphiphils, sowie eine Verwendung des Amphiphils in Zusammensetzungen enthaltend einen Wirkstoff, der in Wasser bei 20 °C zu höchstens 10 g/L löslich ist. Weiterer Gegenstand ist die Verwendung des Amphiphils zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, sowie pflanzliches Vermehrungsmaterial enthaltend das Amphiphil. Kombinationen bevorzugter Merkmale mit anderen bevorzugten Merkmalen werden von der vorliegenden Erfindung umfasst.

In vielen Fällen ist es erforderlich, hydrophobe Wirkstoffe in Wasser zu solubilisieren, ohne den betreffenden Wirkstoff als solchen chemisch zu verändern. Dazu ist es beispielsweise möglich, eine Emulsion herzustellen, wobei sich der betreffende Wirkstoff in der Ölphase der Emulsion befindet. Bei vielen pharmazeutischen Wirkstoffen oder Pflanzenschutzmitteln, insbesondere bei solchen, die mit einer Körperflüssigkeit oder in einem Pflanzensaft transportiert werden sollen, ist ein derartiges Vorgehen jedoch nicht möglich. Unter der Einwirkung von hohen Scherkräften können Emulsionen brechen. Außerdem ist eine Sterilisierung unter Erhalt der Emulsion in vielen Fällen nicht möglich.

Zusammensetzungen enthaltend ein Amphiphil und einen schwerlöslichen Wirkstoff sind allgemein bekannt: WO 2007/125028 offenbart ein Verfahren zur Solubilisierung von hydrophoben Wirkstoffen in wässrigem Medium, wobei man als Hilfsmittel ein hyperverzweigtes Polymer einsetzt, erhältlich durch Herstellung eines hyperverzweigten Polyesters auf Basis einer Dicarbonsäure, Tri- oder Tetracarbonsäure und einem Diol, oder Triol, und Umsetzung des Polyesters mit einer Polyalkylenoxideinheit. WO 2009/021986 offenbart eine Saatgutbeize umfassend einen Wirkstoff und ein hyperverzweigtes Polymer, welches beispielsweise ein hyperverzweigtes Polycarbonat sein kann.

Nachteilig an den bekannten Amphiphilen zur Solubilisierung von hydrophoben Wirkstoffen in wässrigen Medien ist, dass sie nur kleine Mengen an Wirkstoff solubilisieren können. Außerdem sind die Ampphiphile oft selbst nicht wasserlöslich oder nicht wasserdispergierbar, so dass sie zur Solubilisierung in wässrigen Medien nicht geeignet sind. Weiterhin haben Polyester-haltige Amphiphile den Nachteil, dass sie selbst teilweise hydrolyseempfindlich sind, insbesondere durch die vorhandenen terminalen Säuregruppen.

Polycarbonate werden üblicherweise aus der Reaktion von Alkoholen oder Phenolen mit Phosgen oder aus der Reaktion von Alkoholen oder Phenolen mit Dialkyl- oder Diarylcarbonaten erhalten. Technisch bedeutend sind aromatische Polycarbonate, die zum Beispiel aus Bisphenolen hergestellt werden; aliphatische Polycarbonate spielen vom Marktvolumen her gesehen bisher eine untergeordnete Rolle. Die in der Literatur beschriebenen aromatischen oder aliphatischen Polycarbonate sind in der Regel linear oder mit einem lediglich geringen Verzweigungsgrad aufgebaut. Jedoch sind auch hyperverzweigte Polycarbonate allgemein bekannt: WO 2006/089940 (entspricht US 2008/0167430) offenbart wasseremulgierbare hyperverzweigte Polycarbonate, die zumindest teilweise direkt mit einem monofunktionellen Polyalkylenoxidpolyetheralkohol umgesetzt sind. WO 2005/075565 offenbart die Umsetzung eines hyperverzweigten Polycarbonats mit einem Funktionalisierungsreagenz, welches mit den OH- und/oder Carbonat-Gruppen oder Carbamoylgruppen des Polycarbonats reagieren kann. WO 2007/134736 und WO 2008/009516 offenbaren die Umsetzung eines hyperverzweigten Polycarbonats mit einem Funktionalisierungsreagenz, welches mit den OH- und/oder Carbonat-Gruppen oder Carbamoylgruppen des Polycarbonats reagieren kann. Beispielhaft wird die Umsetzung mit Anhydridgruppen enthaltenden Verbindungen genannt, so dass sich Säuregruppen enthaltende Polycarbonate erhalten lassen. US 2009/0030140 offenbart eine thermoplastische Pressform Zusammensetzung enthaltend ein thermoplastischen Polyester und ein hyperverzweigtes Polycarbonat.

Aufgabe der vorliegenen Erfindung war es, ein alternatives Amphiphil zu finden, das zur Solubilisierung von schwerlöslichen Wirkstoffen in wässrigem Medium geeignet ist. Weitere Aufgabe war ein Amphiphil zu finden, dass möglichst hohe Mengen an Wirkstoff, insbesondere agrochemischen Wirkstoff, solubilisieren kann. Des Weiteren sollte das Amphiphil selbst wasserlöslich oder wasserdispergierbar sein. Schließlich war es auch Aufgabe, ein Amphiphil zu finden, das weniger hydrolyseempfindlich ist als Polyester.

Die Aufgabe wurde gelöst durch eine Zusammensetzung enthaltend ein Amphiphil und einen Wirkstoff, der in Wasser bei 20 °C zu höchstens 10 g/L löslich ist, wobei das Amphiphil ein hyperverzweigtes Polycarbonat enthält, das verknüpft ist mit mindestens einem linearen oder Kamm-förmigen Polymer und/oder mindestens einer funktionalen C₁-C₂₄-Einheit enthaltend eine Säuregruppe, eine Aminogruppe oder mindestens zwei Hydroxygruppen,
wobei das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis eines polaren, ethylenisch ungesättigten Monomers, oder
c) ein Polykondensat oder Polyadditionsprodukt enthaltend Polyethylenglykol, oder
d) ein Polyethylenglykol
ist, wobei das Polyethylenglykol d) über einen Linker mit dem Polycarbonat vernüpft ist, und
wobei der Linker ein Polyisocyanat mit einer Funktionalität bezüglich der Isocyanatgruppen von wenigstens 1,5 ist und ausgewählt aus aliphatischen, cycloaliphatischen und aromatischen Di- und Polyisocyanaten sowie Isocyanuraten, Allophanaten, Urethdione und Biurete von aliphatischen, cycloaliphatischen und aromatischen Diisocyanaten,
wobei die funktionalen C₁-C₂₄-Einheit erhältlich ist durch Umsetzung des hyperverzweigten Polycarbonats mit einem zyklischen Carbonsäureanhydrid; oder die funktionalen C₁-C₂₄-Einheit_Hydroxycarbonsäuren, Aminobronsäuren, Hydroxysulfonsäuren, Hydroxysulfate, Aminosulfonsäuren, Aminosulfate, Hydroxyamine, Polyamine oder Polyole sind, die kovalent mittels dem Linker mit dem Poly-carbonat verknüpft sind, und
wobei das Polycarbonat einen Alkohol (B1) enthält, der ein tri- oder höherfunktionelles Polyetherol ist auf Basis von Alkoholen, die mindestens drei OH-Gruppen aufweisen, und C₃-C₂₄ Alkylenoxid.

Der Wirkstoff ist in Wasser bei 20 °C zu höchstens 10 g/L, bevorzugt zu höchstens 2 g/l, besonders bevorzugt zu höchstens 0,5 g/l und speziell zu höchstens 0,1 g/l löslich. Die Zusammensetzung kann einen oder mehrere verschiedene Wirkstoffe enthalten. Beispiele für Wirkstoffe sind agrochemische Wirkstoffe, kosmetische Wirkstoffe, pharmazeutische Wirkstoffe oder Nahrungsergänzungsmittel (wie Vitamine und Carotinoide). Bevorzugte Wirkstoffe sind agrochemische Wirkstoffe.

Beispiele für kosmetische Wirkstoffe sind kosmetische Öle, Riech- und Aromastoffe, Vitamine oder UV-Absorber. Kosmetische Öle sind unter anderem Erdnussöl, Jojobaöl, Kokosnussöl, Mandelöl, Olivenöl, Palmöl, Ricinusöl, Sojaöl oder Weizenkeimöl oder etherische Öle wie Latschenkiefernöl, Lavendelöl, Rosmarinöl, Fichtennadelöl, Kiefernnadelöl, Eukalyptusöl, Pfefferminzöl, Salbeiöl, Bergamotteöl, Terpentinöl, Melissenöl, Wacholderöl, Zitronenöl, Anisöl, Kardamomöl, Campheröl etc. oder deren Mischungen. UV-Absorber sind unter anderem 2-Hydroxy-4-methoxybenzophenon, 2,2',4,4'-Tetrahydroxybenzophenon, 2,2'-Dihydroxy-4,4'-dimethoxybenzophenon, 2,4-Dihydroxybenzophenon, 2-Cyano-3,3-diphenylacrylsäure-2'-ethylhexylester, 2,4,6-Trianilino-p-(carbo-2'-ethylhexyl-1'-oxi)-1,3,5-triazin, 3-(4-Methoxybenzyliden)-cam-pher, N,N-Dimethyl-4-aminobenzoesäure-2-ethylhexylester, Salicylsäure-3,3,5-trimethylcyclohexylester, 4-Isopropyl-dibenzoylmethan, p-Methoxyzimtsäure-2-ethylhexylester und p-Methoxyzimtsäure-2-isoamylester sowie deren Mischungen.

Beispiele für Riech- und Aromastoffe sind wie in der WO 01/49817, oder in "Flavors and Fragrances", Ullmann's Encyclopedia of Industrial Chemistry, Whiley-VCH, 2002 beschrieben, auf die ausdrücklich Bezug genommen wird.

Beispiele für Vitamine sind Vitamine, Provitamine und Vitaminvorstufen aus den Gruppen A, C, E und F, insbesondere 3,4-Didehydroretinol, beta-Carotin (Provitamin des Vitamin A), Ascorbinsaeure (Vitamin C), sowie die Palmitinsaeureester, Glucoside oder Phosphate der Ascorbinsaeure, Tocopherole, insbesondere alpha-Tocopherol sowie seine Ester, z.B. das Acetat, das Nicotinat, das Phosphat und das Succinat; weiterhin Vitamin F, worunter essentielle Fettsaeuren, besonders Linolsaeure, Linolensaeure und Arachidonsaeure, verstanden werden..

Als Beispiele für pharmazeutische Wirkstoffe seien hier genannt: Benzodiazepine, Antihypertensiva, Vitamine, Cytostatika - ins-besondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfo-namide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gal-lenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Der Begriff agrochemische Wirkstoffe (im folgenden auch Pestizide genannt) bezeichnet mindestens einen Wirkstoff ausgewählt aus der Gruppe der Fungizide, Insektizide, Nematizide, Herbizide, Safener und/oder Wachstumsregulatoren. Bevorzugte Pestizide sind Fungizide, Insektizide und Herbizide, insbesondere Fungizide. Auch Mischungen von Pestiziden aus zwei oder mehr der vorgenannten Klassen können verwendet werden. Der Fachmann ist vertraut mit solchen Pestiziden, die beispielsweise in Pesticide Manual, 14th Ed. (2006), The British Crop Protection Council, London, gefunden werden können. Geeignete Insektizide sind Insektizide der Klasse der Carbamate, Organophophate, Organochlor-Insektizide, Phenylpyrazole, Pyrethroide, Neonicotinoide, Spinosine, Avermectine, Milbemycine, Juvenil Hormon Analoga, Alkylhalide, Organozinn-Verbindungen, Nereistoxin-Analoga, Benzoylharnstoffe, Diacylhydrazine, METI Akarizide, sowie Insektizide wie Chloropicrin, Pymetrozin, Flonicamid, Clofentezin, Hexythiazox, Etoxazol, Diafenthiuron, Propargit, Tetradifon, Chlorfenapyr, DNOC, Buprofezin, Cyromazin, Amitraz, Hydramethylnon, Acequinocyl, Fluacrypyrim, Rotenon, oder deren Derivate. Geeignete Fungizide sind Fungizide der Klassen Dinitroaniline, Allylamine, Anilinopyrimidine, Antibiotica, aromatische Kohlenwasserstoffe, Benzenesulfonamide, Benzimidazole, Benzisothiazole, Benzophenone, Benzothiadiazole, Benzotriazine, Benzylcarbamate, Carbamates, Carboxamide, Carbonsäureamdide, Chloronitrile, Cyanoacetamideoxime, Cyanoimidazole, Cyclopropanecarboxamide, Dicarboximide, Dihydrodioxazine, Dinitrophenylcrotonate, Dithiocarbamate, Dithiolane, Ethylphosphonate, Ethylaminothiazolcarboxamide, Guanidines, Hydroxy-(2-amino-)pyrimidine, Hydroxyanilides, Imidazole, Imidazolinone, Anorganika, Isobenzofuranone, Methoxyacrylate, Methoxycarbamates, Morpholines, N-Phenylcarbamate, Oxazolidinedione, Oximinoacetate, Oximinoacetamide, Peptidylpyrimidinnucleoside, Phenylacetamide, Phenylamide, Phenylpyrrole, Phenylharnstoffe, Phosphonate, Phosphorothiolate, Phthalamsäuren, Phthalimide, Piperazine, Piperidine, Propionamide, Pyridazinone, Pyridine, Pyridinylmethylbenzamide, Pyrimidinamine, Pyrimidine, Pyrimidinonehydrazone, Pyrroloquinolinone, Quinazolinone, Chinoline, Chinone, Sulfamide, Sulfamoyltriazole, Thiazolecarboxamide, Thiocarbamate, Thiocarbamate, Thiophanate, Thiophenecarboxamide, Toluamide, Triphenylzinn Verbindungen, Triazine, Triazole. Geeignete Herbizide sind Herbizide der Klassen der Acetamide, Amide, Aryloxyphenoxypropionate, Benzamide, Benzofuran, Benzoesäuren, Benzothiadiazinone, Bipyridylium, Carbamate, Chloroacetamide, Chlorcarbonsäuren, Cyclohexanedione, Dinitroaniline, Dinitrophenol, Diphenylether, Glycine, Imidazolinone, Isoxazole, Isoxazolidinone, Nitrile, N-phenylphthalimide, Oxadiazole, Oxazolidinedione, Oxyacetamide, Phenoxycarbonsäuren, Phenylcarbamate, Phenylpyrazole, Phenylpyrazoline, Phenylpyridazine, Phosphinsäuren, Phosphoroamidate, Phosphorodithioate, Phthalamate, Pyrazole, Pyridazinone, Pyridine, Pyridincarbonsäuren, Pyridinecarboxamide, Pyrimidindione, Pyrimidinyl(thio)benzoate, Chinolincarbonsäuren, Semicarbazone, Sulfonylaminocarbonyltriazolinone, Sulfonylharnstoffe, Tetrazolinone, Thiadiazole, Thiocarbamate, Triazine, Triazinone, Triazole, Triazolinone, Triazolinone, Triazolocarboxamide, Triazolopyrimidine, Triketone, Uracile, Harnstoffe.

In einer Ausführungsform enthält das Pestizid ein Insektizid, bevorzugt besteht das Pestizid aus mindestens einem Insektizid. Bevorzugte Insektizide sind Fipronil, Allethrin, Alpha-Cypermethrin, Beta-Cyfluthrin, Bifenthrin, Bioallethrin, 4-Chlor-2-(2-chlor-2methylpropyl)-5-[(6-iod-3-pyridinyl)methoxy]-3(2H)-pyridazinone (CAS-RN: 120955-77-3), Chlorfenapyr, Chlorpyrifos, Cyfluthrin, Cyhalothrin, Cypermethrin, Deltamethrin, Etofenprox, Fenoxycarb, Flufenoxuron, Hydramethylnon, Metaflumizone, Permethrin, Pyriproxifen, Silafluofen, Tebufenozide und Tralomethrin. Besonders bevorzugte Insektizide sind Fipronil, Alpha-Cypermethrin, Bifenthrin, Chlorfenapyr, Cyfluthrin, Cypermethrin, Deltamethrin, Etofenprox, Hydramethylnon, Metaflumizone, Permethrin. Ganz besonders bevorzugte Insektizide sind Fipronil, Alpha-Cypermethrin, Deltamethrin, Chlorfe-napyr, Hydramethylnon und Metaflumizone. Insbesondere bevorzugtes Insektizid ist Fipronil. In einer weiteren Ausführungsform enthält das Pestizid ein Fungizid, bevorzugt besteht das Pestizid aus mindestens einem Fungizid. Bevorzugte Fungizide sind Pyraclostrobin, Metconazol und Epoxiconazol. In einer weiteren Ausführungsform enthält das Pestizid ein Herbizid, bevorzugt besteht das Pestizid aus mindestens einem Herbizid. In einer weiteren Ausführungsform enthält das Pestizid ein Wachstumsregulator, bevorzugt besteht das Pestizid aus mindestens einem Wachstumsregulator.

Die erfindungsgemäße Zusammensetzung umfasst üblicherweise 0.1 bis 70 Gew.% Wirkstoff, bevorzugt 1 bis 50 Gew. %, insbesondere 3 bis 30 Gew.%, bezogen auf die Zusammensetzung.

Amphiphile enthalten üblicherweise mindestens einen polaren (hydrophilen) und mindestens einen apolaren (hydrophoben) Teil. Typische Amphiphile sind Fettsäuren, Tenside und Phospholipide. Die Zusammensetzung kann ein oder mehrere verschiedene Amphiphile enthalten.

Unter hyperverzweigten Polycarbonaten werden im Rahmen dieser Erfindung unvernetzte Makromoleküle mit Hydroxyl- und Carbonat- oder Carbamoylchloridgruppen verstanden, die sowohl strukturell als auch molekular uneinheitlich sind. Sie können auf der einen Seite ausgehend von einem Zentralmolekül analog zu Dendrimeren, jedoch im Unterschied zu diesen mit uneinheitlicher Kettenlänge der Äste aufgebaut sein. Hyperverzweigte Polymere sind daher zu unterscheiden von Dendrimeren (US 6,399,048). Im Sinne der vorliegenden Erfindung umfassen hyperverzweigte Polymere keine Dendrimere. Die hyperverzweigte Polymere können auf der anderen Seite auch linear, mit funktionellen, verzweigten Seitengruppen, aufgebaut sein oder aber, als Kombination der beiden Extreme, lineare und verzweigte Molekülteile aufweisen. Zur Definition von Dendrimeren und hyperverzweigten Polymeren siehe auch P.J. Flory, J. Am. Chem. Soc. 1952, 74, 2718 und H. Frey et al., Chem. Eur. J. 2000, 6, 2499.

Unter "hyperverzweigt" wird im Zusammenhang mit der vorliegenden Erfindung verstanden, dass der Verzweigungsgrad (Degree of Branching, DB), das heißt der Quotient aus der Summe der mittleren Anzahl dendritischer Verknüpfungen plus mittlere Anzahl der Endgruppen und der Summe der mittleren Anzahl dendritischer und linearer Verknüpfungen plus der mittleren Anzahl der Endgruppen pro Molekül multipliziert mit 100, 10 bis 99,9 %, bevorzugt 20 bis 99 %, besonders bevorzugt 20 bis 95 % beträgt. Unter "dendrimer" wird im Zusammenhang mit der vorliegenden Erfindung verstanden, daß der Verzweigungsgrad 99,9 - 100% beträgt. Zur Definition des "Degree of Branching" siehe H. Frey et al., Acta Polym. 1997, 48, 30.

Es stellt einen Vorteil der vorliegenden Erfindung dar, daß die erfindungsgemäßen Polycarbonate unvernetzt sind. "Unvernetzt" im Rahmen dieser Schrift bedeutet, daß ein Vernetzungsgrad von weniger als 15 Gew.%, bevorzugt von weniger als 10 Gew.%, bestimmt über den unlöslichen Anteil des Polymeren, vorhanden ist. Der unlösliche Anteil des Polymeren wurde bestimmt durch vierstündige Extraktion mit dem gleichen Lösungsmittel, wie es für die Gelpermeationschromatographie zur Bestimmung der Molekulargewichtsverteilung der Polymere verwendet wird, also Tetrahydrofuran, Dimethylacetamid oder Hexafluorisopropanol, je nachdem, in welchem Lösungsmittel das Polymer besser löslich ist, in einer Soxhlet-Apparatur und nach Trocknung des Rückstandes bis zur Gewichtskonstanz Wägung des verbliebenen Rückstandes.

Das hyperverzweigte Polycarbonat ist üblicherweise erhältlich durch
a) Herstellung eines Kondensationsproduktes (K) durch Umsetzung eines organisches Carbonats (A) oder eines Phosgenderivates mit einem Alkohol (B1), der mindestens drei Hydroxygruppen aufweist, und
b) intermolekularer Umsetzung von K zu dem hyperverzweigten Polycarbonat, wobei das Mengenverhältnis der OH-Gruppen zu den Carbonat- oder Phosgengruppen so gewählt wird, dass K im Mittel entweder i) eine Carbonat- oder Carbamoylchloridgruppe und mehr als eine OH-Gruppe, oder ii) eine OH-Gruppe und mehr als eine Carbonat- oder Carbamoylgruppe aufweisen. Bevorzugt wird das Polycarbonat auf diese Weise erhalten.

Zur Herstellung des Kondensationsproduktes (K) kann ein organisches Carbonat (A) oder ein Phosgenderivat verwendet werden. Geeignete Phosgenderivate sind beispielsweise Phosgen, Diphosgen oder Triphosgen, bevorzugt Phosgen. Bevorzugt wird ein organisches Carbonat eingesetzt.

Bei den Resten R der als Ausgangsmaterial eingesetzten organischen Carbonate (A) der allgemeinen Formel RO[(CO)O]ₙR handelt es sich jeweils unabhängig voneinander um einen geradkettigen oder verzweigten aliphatischen, aromatisch/aliphatischen (araliphatischen) oder aromatischen Kohlenwasserstoffrest mit 1 bis 20 C-Atomen. Die beiden Reste R können auch unter Bildung eines Ringes miteinander verbunden sein. Die beiden Reste R können gleich oder unterschiedlich sein, bevorzugt sind sie gleich. Bevorzugt handelt es sich um einen aliphatischen Kohlenwasserstoffrest und besonders bevorzugt um einen geradkettigen oder verzweigten Alkylrest mit 1 bis 5 C-Atomen, oder um einen substituierten oder unsubstituierten Phenylrest. R steht dabei für einen geradkettigen oder verzweigten, bevorzugt geradkettigen, (cyclo)aliphatischen, aromatisch/aliphatischen oder aromatischen, bevorzugt (cyclo)aliphatischen oder aromatischen, besonders bevorzugt aliphatischen Kohlenwasserstoffrest mit 1 bis 20 C-Atomen, bevorzugt 1 bis 12, besonders bevorzugt 1 bis 6 und ganz besonders bevorzugt 1 bis 4 Kohlenstoffatomen. Beispiele dafür sind Methyl, Ethyl, iso-Propyl, n-Propyl, n-Butyl, iso-Butyl, sek-Butyl, tert-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, o- oder p-Tolyl oder Naphthyl. Bevorzugt sind Methyl, Ethyl, n-Butyl und Phenyl. Die Reste R können dabei gleich oder verschieden sind, bevorzugt sind sie gleich. Die Reste R können auch unter Bildung eines Ringes miteinander verbunden sein. Beispiele für derartige zweibindige Reste R sind 1,2-Ethylen, 1,2-Propylen und 1,3-Propylen. Generell handelt es sich bei n um eine ganze Zahl von 1 bis 5, bevorzugt von 1 bis 3, besonders bevorzugt von 1 bis 2. Bei den Carbonaten kann es sich bevorzugt um einfache Carbonate der allgemeinen Formel RO(CO)OR handeln, d.h. in diesem Falle steht n für 1.

Beispiele geeigneter Carbonate umfassen aliphatische, aromatisch/aliphatische oder aromatische Carbonate wie Ethylencarbonat, 1,2- oder 1,3-Propylencarbonat, Diphenylcarbonat, Ditolylcarbonat, Dixylylcarbonat, Dinaphthylcarbonat, Ethylphenylcarbonat, Dibenzylcarbonat, Dimethylcarbonat, Diethylcarbonat, Di-n-propylcarbonat, Di-nbutylcarbonat, Diisobutylcarbonat, Dipentylcarbonat, Dihexylcarbonat, Dicyclohexylcarbonat, Diheptylcarbonat, Dioctylcarbonat, Didecylcarbonat oder Didodecylcarbonat. Beispiele für Carbonate, bei denen n größer als 1 ist, umfassen Dialkyldicarbonate, wie Di(tert.butyl)dicarbonat oder Dialkyltricarbonate wie Di(tert.butyl)tricarbonat. Ein bevorzugtes aromatisches Carbonat ist Diphenylcarbonat. Bevorzugt werden aliphatische Carbonate eingesetzt, insbesondere solche, bei denen die Reste 1 bis 5 C-Atome umfassen, wie zum Beispiel Dimethylcarbonat, Diethylcarbonat, Di-n-propylcarbonat, Di-n-butylcarbonat oder Diisobutylcarbonat. Speziell bevorzugt wird Diethylcarbonat.

Das hyperverzweigte Polycarbonat enthält einen Alkohol (B1), der ein tri- oder höherfunktionelles Polyetherol ist auf Basis von Alkoholen, die mindestens drei OH-Gruppen aufweisen, und C₃-C₂₄ Alkylenoxid. Geeignete Alkohole, die mindestens drei OH-Gruppen aufweisen, sind wie oben beschrieben, bevorzugt Glycerin, Trimethylolethan, Trimethylolpropan, 1,2,4-Butantriol, 1,2,3-Hexantriol, 1,2,4-Hexantriol, Pentaerythrit, besonders bevorzugt Glycerin oder Trimethylolpropan. Bevorzugte C₃-C₂₄ Alkylenoxide sind unter anderem Propylenoxid, Butylenoxid, Pentylenoxid und deren Mischungen, besonders bevorzugt Propylenoxid. Die tri- oder höherfunktionellen Polyetherole enthalten meist mindestens ein bis 30, bevorzugt zwei bis 30, besonders bevorzugt drei bis 20 Moleküle C₃-C₂₄ Alkylenoxid in polymerisierter Form. Besonders bevorzugter Alkohol (B1) ist ein trifunktionelles Polyetherol auf Basis von Glycerin, Trimethylolethan, Trimethylolpropan, 1,2,4-Butantriol und/oder Pentaerythrit, und Propylenoxid, wobei das Polyetherol mindestens drei, bevorzugt drei bis 30, besonders bevorzugt drei bis 20 Moleküle Propylenoxid in polymerisierter Form enthält.

Zusätzlich zu dem Alkohol (B1) kann das Polycarbonat einen difunktionellen Alkohol (B2) als Aufbaukomponente aufweisen, mit der Maßgabe, dass die mittlere OH-Funktionalität aller eingesetzten Alkohole B zusammen größer als 2 ist. Die Alkohole (B1) und (B2) werden hier zusammen als (B) bezeichnet. Geeignete difunktionelle Alkohole B2 sind unter anderem Diethylenglykol, Triethylenglykol, 1,2- und 1,3-Propandiol, Dipropylenglykol, Tripropylenglykol, Neopentylglykol, 1,2-, 1,3- und 1,4-Butandiol, 1,2-, 1,3- und 1,5-Pentandiol, 1,6-Hexandiol, 1,2- oder 1,3-Cyclopentandiol, 1,2-, 1,3- oder 1,4-Cyclohexandiol, 1,1-, 1,2-, 1,3- oder 1,4-Cyclo-hexandimethanol, Bis(4-Hydroxycyclohexyl)methan, Bis(4-Hydroxycyclo-hexyl)ethan, 2,2- Bis(4-Hydroxycyclohekyl)propan, 1,1'-Bis(4-Hydroxyphenyl)-3,3-5-trimethylcyclo-hexan, Resorcin, Hydrochinon, 4,4'-Dihydroxydiphenyl, Bis-(4-Hydroxyphenyl)sulfid, Bis(4-Hydroxyphenyl)sulfon, Bis(hydroxymethyl)benzol, Bis(Hydroxymethyl)toluol, Bis(p-hydroxyphenyl)methan, Bis(p-hydroxyphenyl)ethan, 2,2-Bis(p-hydroxyphe-nyl)propan, 1,1-Bis(p-hydroxyphenyl)cyclohexan, Dihydroxybenzophenon, difunktionelle Polyetherpolyole auf Basis Ethylenoxid, Propylenoxid, Butylenoxid oder deren Gemische, Polytetrahydrofuran mit einem Molgewicht von 162 bis 2000, Polycaprolacton oder Polyesterole auf Basis von Diolen und Dicarbonsäuren. Bevorzugter difunktioneller Alkohol (B2) sind difunktionelle Polyetherpolyole auf Basis Ethylenoxid, Propylenoxid, Butylenoxid oder deren Gemische, und Polyesterole auf Basis von Diolen und Dicarbonsäuren.

Die Diole dienen zur Feineinstellung der Eigenschaften des Polycarbonates. Falls difunktionelle Alkohole eingesetzt werden, wird das Verhältnis von difunktionellen Alkoholen (B2) zu den mindestens trifunktionellen Alkoholen (B1) vom Fachmann je nach den gewünschten Eigenschaften des Polycarbonates festgelegt. Im Regelfalle beträgt die Menge des oder der Alkohole (B2) 0 bis 50 mol % bezüglich der Gesamtmenge aller Alkohole (B1) und (B2) zusammen. Bevorzugt beträgt die Menge 0 bis 35 mol %, besonders bevorzugt 0 bis 25 mol % und ganz besonders bevorzugt 0 bis 10 mol %.

Die Reaktion von Phosgen, Diphosgen oder Triphosgen mit dem Alkohol oder Alkoholgemisch erfolgt in der Regel unter Eliminierung von Chlorwasserstoff, die Reaktion der Carbonate mit dem Alkohol oder Alkoholgemisch zum erfindungsgemäßen hochfunktionellen hochverzweigten Polycarbonat erfolgt unter Eliminierung des monofunktionellen Alkohols oder Phenols aus dem Carbonat-Molekül.

Das hyperverzweigte Polycarbonat ist nach dieser Reaktion, also ohne weitere Modifikation, hochfunktionell mit Hydroxylgruppen und mit Carbonatgruppen beziehungsweise Carbamoylchloridgruppen terminiert. Unter einem hochfunktionellen Polycarbonat ist im Rahmen dieser Erfindung ein Produkt zu verstehen, das neben den Carbonatgruppen, die das Polymergerüst bilden, end- oder seitenständig weiterhin mindestens drei, bevorzugt mindestens vier, mehr bevorzugt mindestens sechs funktionelle Gruppen aufweist. Bei den funktionellen Gruppen handelt es sich um Carbonatgruppen beziehungsweise Carbamoylchloridgruppen und/oder um OH-Gruppen. Die Anzahl der end- oder seitenständigen funktionellen Gruppen ist prinzipiell nach oben nicht beschränkt, jedoch können Produkte mit sehr hoher Anzahl funktioneller Gruppen unerwünschte Eigenschaften, wie beispielsweise hohe Viskosität oder schlechte Löslichkeit, aufweisen. Die hochfunktionellen Polycarbonate der vorliegenden Erfindung weisen zumeist nicht mehr als 500 end- oder seitenständige funktionelle Gruppen, bevorzugt nicht mehr als 100 end- oder seitenständige funktionelle Gruppen auf.

Bei der Herstellung der hochfunktionellen Polycarbonate ist es notwendig, das Verhältnis von den OH-Gruppen enthaltenden Verbindungen zu Phosgen oder Carbonat (A) so einzustellen, dass das resultierende einfachste Kondensationsprodukt (im Weiteren Kondensationsprodukt (K) genannt) im Mittel entweder i) eine Carbonat- oder Carbamoylchloridgruppe und mehr als eine OH-Gruppe oder ii) eine OH-Gruppe und mehr als eine Carbonat- oder Carbamoylchloridgruppe, bevorzugt im Mittel entweder i) eine Carbonat- oder Carbamoylchloridgruppe und mindestens zwei OH-Gruppen oder ii) eine OH-Gruppe und mindestens zwei Carbonat- oder Carbamoylchloridgruppen enthält.

Es kann ferner sinnvoll sein, zur Feineinstellung der Eigenschaften des Polycarbonates mindestens eine zweiwertige carbonylreaktive Verbindung (A1) einzusetzen. Darunter werden solche Verbindungen verstanden, die zwei Carbonat- und/oder Carboxylgruppen aufweisen. Carboxylgruppen können dabei Carbonsäuren, Carbonsäurechloride, Carbonsäureanhydride oder Carbonsäureester sein, bevorzugt Carbonsäureanhydride oder Carbonsäureester und besonders bevorzugt Carbonsäureester. Falls solche zweiwertigen Verbindungen (A1) eingesetzt werden, so wird das Verhältnis von (A1) zu den Carbonaten bzw. Phosgenen (A) vom Fachmann je nach den gewünschten Eigenschaften des Polycarbonates festgelegt. Im Regelfalle beträgt die Menge des oder der zweiwertigen Verbindung (A1) 0 bis 40 mol % bezüglich der Gesamtmenge aller Carbonate/Phosgene (A) und Verbindungen (A1) zusammen. Bevorzugt beträgt die Menge 0 bis 35 mol %, besonders bevorzugt 0 bis 25 mol % und ganz besonders bevorzugt 0 bis 10 mol %. Beispiele für Verbindungen (A1) sind Dicarbonate oder Dicarbamoylchloride von Diolen, beispielsweise Ethylenglykol, 1,2-Propandiol, 1,3-Propandiol, 1,1-Dimethylethan-1,2-diol, 2-Butyl-2-ethyl-1,3-Propandiol, 2-Ethyl-1,3-Propandiol, 2-Methyl-1,3-Propan-diol, Neopentylglykol, Hydroxypivalinsäureneopentylglykolester, 1,2-, 1,3- oder 1,4-Butandiol, 1,6-Hexandiol, 1,10-Dekandiol, Bis-(4-hydroxycyclo-hexan)isopropyliden, Tetramethylcyclobutandiol, 1,2-, 1,3- oder 1,4-Cyclohexandiol, Cyclooctandiol, Norbornandiol, Pinandiol, Decalindiol, 2-Ethyl-1,3-Hexandiol, 2,4-Diethyl-oktan-1,3-diol, Hydrochinon, Bisphenol A, Bisphenol F, Bisphenol B, Bisphenol S, 2,2-Bis(4-hydroxy-cyclohexyl)propan, 1,1-, 1,2-, 1,3- und 1,4-Cyclohexandimethanol, 1,2-, 1,3- oder 1,4-Cyclohexandiol. Diese können beispielsweise hergestellt werden, indem man diese Diole mit einem Überschuß an beispielsweise den oben aufgeführten Carbonaten RO(CO)OR oder Chlorkohlensäureestern umsetzt, so dass die so erhaltenen Dicarbonate beidseitig mit Gruppen RO(CO)- substituiert sind. Eine weitere Möglichkeit ist die, die Diole zunächst mit Phosgen zu den korrespondierenden Chlorkohlensäureestern der Diole umzusetzen und anschließend mit Alkoholen umzusetzen.

Weitere Verbindungen (A1) sind Dicarbonsäuren, Ester von Dicarbonsäuren, bevorzugt die Methyl-, Ethyl-, iso-Propyl-, n-Propyl-, n-Butyl-, iso-Butyl-, sek-Butyl- oder tert-Butylester, besonders bevorzugt die Methyl-, Ethyl- oder n-Butylester. Beispiele für derartige Dicarbonsäuren sind Oxalsäure, Maleinsäure, Fumarsäure, Bernsteinsäure, Glutarsäure, Adipinsäure, Sebacinsäure, Dodekandisäure, o-Phthal-säure, Isophthalsäure, Terephthalsäure, Azelainsäure, 1,4-Cyclohexandicarbonsäure oder Tetrahydrophthalsäure, Korksäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, dimere Fettsäuren, deren Isomere und Hydrierungsprodukte.

Die einfachste Struktur des Kondensationsproduktes (K), dargestellt am Beispiel der Umsetzung eines Carbonats (A) mit einem Di- oder Polyalkohol (B) ergibt dabei die Anordnung XYₘ oder YₘX, wobei X eine Carbonat- oder Carbamoylgruppe, Y eine Hydroxyl-Gruppe und m in der Regel eine ganze Zahl größer 1 bis 6, vorzugsweise größer 1 bis 4, besonders bevorzugt größer 1 bis 3 darstellt. Die reaktive Gruppe, die dabei als einzelne Gruppe resultiert, wird im folgenden generell "fokale Gruppe" genannt.

Liegt beispielsweise bei der Herstellung des einfachsten Kondensationsproduktes (K) aus einem Carbonat und einem zweiwertigen Alkohol das molare Umsetzungsverhältnis bei 1:1, so resultiert im Mittel ein Molekül des Typs XY, veranschaulicht durch die allgemeine Formel (I).

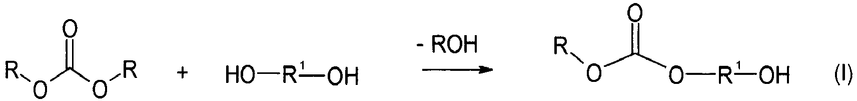

Bei der Herstellung des Kondensationsproduktes (K) aus einem Carbonat und einem dreiwertigen Alkohol bei einem molaren Umsetzungsverhältnis von 1 : 1 resultiert im Mittel ein Molekül des Typs XY₂, veranschaulicht durch die allgemeine Formel (II). Fokale Gruppe ist hier eine Carbonatgruppe.

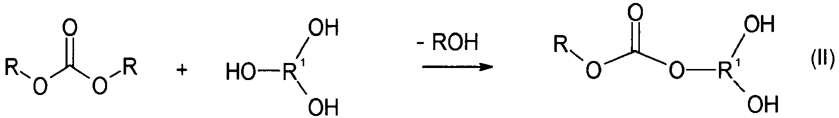

Bei der Herstellung des Kondensationsproduktes (K) aus einem Carbonat und einem vierwertigen Alkohol ebenfalls mit dem molaren Umsetzungsverhältnis 1 : 1 resultiert im Mittel ein Molekül des Typs XY₃, veranschaulicht durch die allgemeine Formel (III). Fokale Gruppe ist hier eine Carbonatgruppe.

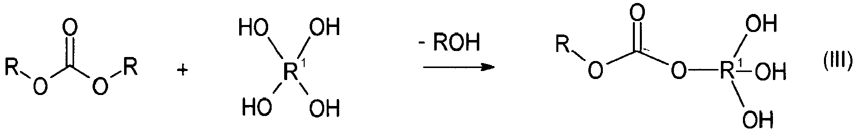

In den Formeln (I) bis (III) hat R die eingangs definierte Bedeutung und R¹steht für einen aliphatischen oder aromatischen Rest.

Weiterhin kann die Herstellung des Kondensationsprodukts (K) zum Beispiel auch aus einem Carbonat und einem dreiwertigen Alkohol, veranschaulicht durch die allgemeine Formel (IV), erfolgen, wobei das Umsetzungsverhältnis bei molar 2:1 liegt. Hier resultiert im Mittel ein Molekül des Typs X₂Y, fokale Gruppe ist hier eine OH-Gruppe. In der Formel (IV) haben R und R¹ die gleiche Bedeutung wie oben in den Formeln (I) bis (III).

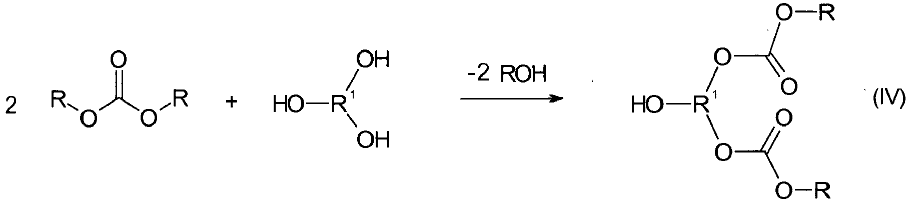

Werden zu den Komponenten zusätzlich difunktionelle Verbindungen, z.B ein Dicarbonat oder ein Diol gegeben, so bewirkt dies eine Verlängerung der Ketten, wie beispielsweise in der allgemeinen Formel (V) veranschaulicht. Es resultiert wieder im Mittel ein Molekül des Typs XY₂, fokale Gruppe ist eine Carbonatgruppe.

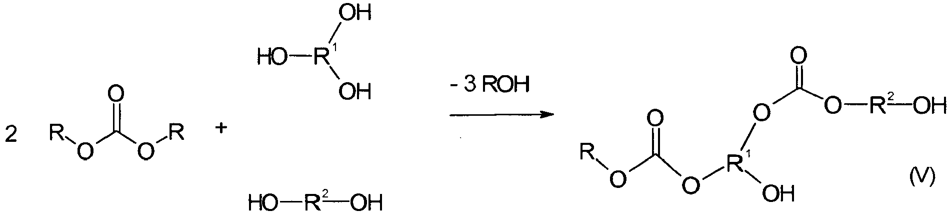

In Formel (V) bedeutet R² einen aliphatischen oder aromatischen Rest, R und R¹ sind wie vorstehend beschrieben definiert.

Es können auch mehrere Kondensationsprodukte (K) zur Synthese eingesetzt werden. Hierbei können einerseits mehrere Alkohole beziehungsweise mehrere Carbonate eingesetzt werden. Weiterhin lassen sich durch die Wahl des Verhältnisses der eingesetzten Alkohole und der Carbonate bzw. der Phosgene Mischungen verschiedener Kondensationsprodukte unterschiedlicher Struktur erhalten. Dies sei am Beispiel der Umsetzung eines Carbonates mit einem dreiwertigen Alkohol beispielhaft erläutert. Setzt man die Ausgangsprodukte im Verhältnis 1:1 ein, wie in (II) dargestellt, so erhält man ein Molekül XY₂. Setzt man die Ausgangsprodukte im Verhältnis 2:1 ein, wie in (IV) dargestellt, so erhält man ein Molekül X₂Y. Bei einem Verhältnis zwischen 1:1 und 2:1 erhält man eine Mischung von Molekülen XY₂ und X₂Y.

Typische Reaktionsbedingungen der Umsetzung von (A) mit (B) zum Kondensationsprodukt (K) sind im folgenden dargestellt:
Die Stöchiometrie der Komponenten (A) und (B) wird generell so gewählt, daß das resultierende Kondensationsprodukt (K) entweder eine Carbonat- oder Carbamoylchloridgruppe und mehr als eine OH-Gruppe oder eine OH-Gruppe und mehr als eine Carbonat- oder Carbamoylchloridgruppe aufweist. Dies wird im ersten Fall erreicht durch eine Stöchiometrie von 1 mol Carbonatgruppen : >2 mol OH-Gruppen, beispielsweise eine Stöchiometrie von 1 : 2,1 bis 8, bevorzugt 1 : 2,2 bis 6, besonders bevorzugt 1 : 2,5 bis 4 und ganz besonders bevorzugt 1 : 2,8 bis 3,5. Im zweiten Fall wird dies erreicht durch eine Stöchiometrie von mehr als 1 mol Carbonatgruppen : <1 mol OH-Gruppen, beispielsweise eine Stöchiometrie von 1 : 0,1 bis 0,48, bevorzugt 1 : 0,15 bis 0,45, besonders bevorzugt 1 : 0,25 bis 0,4 und ganz besonders bevorzugt 1 : 0,28 bis 0,35.

Die Temperatur sollte ausreichend für die Umsetzung des Alkohols mit der entsprechenden Carbonylkomponente sein. In der Regel ist für die Umsetzung mit einem Phosgen eine Temperatur von -20 °C bis 120 °C, bevorzugt 0 bis 100 und besonders bevorzugt 20 bis 80 °C ausreichend. Bei Einsatz eines Carbonats sollte die Temperatur 60 bis 280 °C, bevorzugt 80 bis 250 °C, besonders bevorzugt 100 bis 250 und ganz besonders bevorzugt 120 bis 250 °C betragen.

Die Herstellung erfolgt zumeist in einem Druckbereich von 0,1 mbar bis 20 bar, bevorzugt bei 1 mbar bis 5 bar, in Reaktoren oder Reaktorkaskaden, die im Batchbetrieb, halbkontinuierlich oder kontinuierlich betrieben werden.

Als Lösungsmittel kommen aromatische und/oder (cyclo)aliphatische Kohlenwasserstoffe und deren Gemische, halogenierte Kohlenwasserstoffe, Ketone, Ester und Ether in Frage, bevorzugt Butylacetat, Dimethylformamid, Dimethylacetamid, Dimethylsulfoxid, Methoxypropylacetat, iso-Butylmethylketon, 2-Butanon, aromatische Kohlenwasserstoffe (wie Solvesso®-Marken), Cyclohexan, Chlorbenzol und Xylol. Es stellt eine bevorzugte Ausführungsform dar, die Reaktion ohne Lösungsmittel durchzuführen.

Die Reihenfolge der Zugabe der einzelnen Komponenten spielt zumeist eine untergeordnete Rolle. In der Regel ist es sinnvoll die Überschußkomponente der beiden Reaktionspartner vorzulegen und die Unterschußkomponente zuzugeben. Alternativ ist es ebenfalls möglich, die beiden Komponenten vor Reaktionsbeginn miteinander zu vermischen und diese Gemisch anschließend auf die erforderliche Reaktionstemperatur aufzuheizen.

Die beispielhaft in den Formeln (I) - (V) beschriebenen einfachen Kondensationsprodukte (K) reagieren erfindungsgemäß bevorzugt sofort intermolekular weiter unter Bildung von hochfunktionellen Polykondensationsprodukten, im folgenden Polykondensationsprodukte (P) genannt. Die Umsetzung zum Kondensationsprodukt (K) und zum Polykondensationsprodukt (P) erfolgt üblicherweise bei einer Temperatur von 0 bis 300 °C, bevorzugt 0 bis 250°C, besonders bevorzugt bei 60 bis 250°C und ganz besonders bevorzugt bei 80 bis 250°C in Substanz oder in Lösung. Dabei können allgemein alle Lösungsmittel verwendet werden, die gegenüber den jeweiligen Edukten inert sind. Bevorzugt verwendet werden organische Lösungsmittel, wie zum Beispiel die oben genannten und besonders bevorzugt Decan, Dodecan, Cyclohexan, Benzol, Toluol, Chlorbenzol, Xylol, Dimethylformamid, Dimethylacetamid, Dimethylsulfoxid oder Solventnaphtha. In einer bevorzugten Ausführungsform wird die Kondensationsreaktion in Substanz durchgeführt. Der bei der Reaktion freiwerdende monofunktionelle Alkohol oder das Phenol ROH kann zur Beschleunigung der Reaktion aus dem Reaktionsgleichgewicht entfernt werden, zum Beispiel destillativ, gegebenenfalls bei vermindertem Druck.

Die Abtrennung des Alkohols oder Phenols kann auch durch Durchleiten eines unter den Reaktionsbedingungen im wesentlichen inerten Gasstromes (Strippen), wie z.B. Stickstoff, Wasserdampf, Kohlenstoffdioxid oder auch eines sauerstoffhaltigen Gases, wie z.B. Luft oder Magerluft, unterstützt werden. Falls Abdestillieren vorgesehen ist, ist es regelmäßig empfehlenwert, solche Carbonate einzusetzen, welche bei der Umsetzung Alkohole oder Phenole ROH mit einem Siedepunkt von weniger als 140°C bei dem vorliegenden Druck freisetzen. Alternativ können die freigesetzten Alkohole durch azeotrope Destillation mittels Schleppmittel (z.B. Toluol, Xylol, Chlorbenzol, Cyclohexan) oder durch Anlegen eines Vakuums entfernt und damit die Bildung des Polykondensats unterstützt werden.

Zur Beschleunigung der Reaktion können auch Katalysatoren oder Katalysatorgemische zugegeben werden. Geeignete Katalysatoren sind Verbindungen, die Veresterungs- oder Umesterungsreaktionen katalysieren, zum Beispiel Alkalihydroxide, Alkalicarbonate, Alkalihydrogencarbonate, vorzugsweise des Natriums, Kaliums oder Cäsiums, tertiäre Amine, Guanidine, Ammoniumverbindungen, Phosphoniumverbindungen, Aluminium-, Zinn-, Zink, Titan-, Zirkon- oder Wismut-organische Verbindungen, weiterhin sogenannte Doppelmetallcyanid (DMC)-Katalysatoren, wie zum Beispiel in der DE 10138216 oder in der DE 10147712 beschrieben. Vorzugsweise werden Kaliumhydroxid, Kaliumcarbonat, Kaliumhydrogencarbonat, Diazabicyclooctan (DABCO), Diazabicyclononen (DBN), Diazabicycloundecen (DBU), Imidazole, wie Imidazol, 1-Methylimidazol oder 1,2-Dimethylimidazol, Titan-tetra-butylat, Titantetraisopropylat, Dibutylzinnoxid, Dibutylzinn-dilaurat, Zinndioctoat, Zirkonacetylacetonat oder Gemische davon eingesetzt. Die Zugabe des Katalysators erfolgt im allgemeinen in einer Menge von 50 bis 10000, bevorzugt von 100 bis 5000 Gew. ppm bezogen auf die Menge des eingesetzten Alkohols oder Alkoholgemisches. Gegebenenfalls kann es erforderlich sein, den Katalysator in geringen Mengen eines geeigneten Lösungsmittels vorzulösen.

Ferner ist es auch möglich, sowohl durch Zugabe des geeigneten Katalysators, als auch durch Wahl einer geeigneten Temperatur die intermolekulare Polykondensationsreaktion zu steuern. Weiterhin lässt sich über die Zusammensetzung der Ausgangskomponenten und über die Verweilzeit das mittlere Molekulargewicht des Polymeren (P) einstellen.

Die Kondensationsprodukte (K) bzw. die Polykondensationsprodukte (P), die bei erhöhter Temperatur hergestellt wurden, sind bei Raumtemperatur üblicherweise über einen längeren Zeitraum, beispielsweise über mindestens 6 Wochen, stabil, ohne Trübungen, Ausfällungen und/oder einen Viskositätsanstieg zu zeigen. Aufgrund der Beschaffenheit der Kondensationsprodukte (K) ist es möglich, daß aus der Kondensationsreaktion Polykondensationsprodukte (P) mit unterschiedlichen Strukturen resultieren können, die Verzweigungen, aber keine Vernetzungen aufweisen. Ferner weisen die Polykondensationsprodukte (P) im Idealfall entweder eine Carbonat- oder Carbamoylchloridgruppe als fokale Gruppe und mehr als zwei OH-Gruppen oder aber eine OH-Gruppe als fokale Gruppe und mehr als zwei Carbonat- oder Carbamoylchloridgruppen auf. Die Anzahl der reaktiven Gruppen ergibt sich dabei aus der Beschaffenheit der eingesetzten Kondensationsprodukte (K) und dem Polykondensationsgrad.

Beispielsweise kann ein Kondensationsprodukt (K) gemäß der allgemeinen Formel (II) durch dreifache intermolekulare Kondensation zu zwei verschiedenen Polykondensationsprodukten (P), die in den allgemeinen Formeln (VI) und (VII) wiedergegeben werden, reagieren.

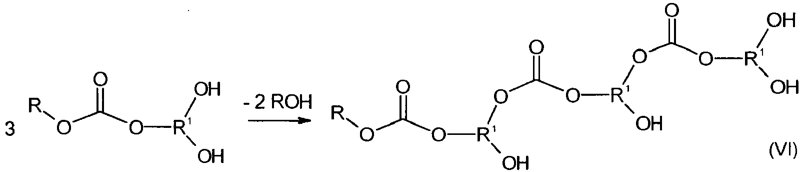

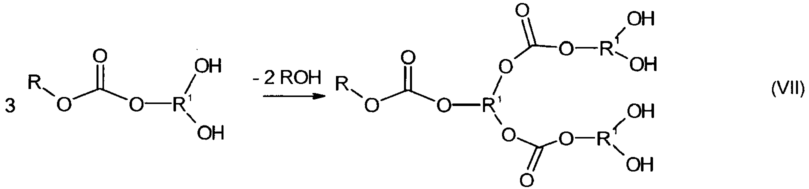

In Formel (VI) und (VII) sind R und R¹ wie vorstehend definiert.

Zum Abbruch der intermolekularen Polykondensationsreaktion gibt es verschiedene Möglichkeiten. Beispielsweise kann die Temperatur auf einen Bereich abgesenkt werden, in dem die Reaktion zum Stillstand kommt und das Produkt (K) oder das Polykondensationsprodukt (P) lagerstabil ist. Dies ist in der Regel unterhalb von 60 °C, bevorzugt unter 50 °C, besonders bevorzugt unterhalb von 40 °C und ganz besonders bevorzugt bei Raumtemperatur der Fall. Weiterhin kann man den Katalysator desaktivieren, bei basischen Katalysatoren zum Beispiel durch Zugabe einer sauren Komponente, zum Beispiel einer Lewis-Säure oder einer organischen oder anorganischen Protonensäure. Ferner ist es möglich, die Reaktion durch Verdünnen mit einem vorgekühlten Lösungsmittel zu stoppen. Dies ist insbesondere dann bevorzugt, wenn man die Viskosität des Reaktionsgemischs durch Zugabe von Lösungsmittel anpassen muß.

In einer weiteren Ausführungsform kann, sobald aufgrund der intermolekularen Reaktion des Kondensationsproduktes (K) ein Polykondensationsprodukt (P) mit gewünschten Polykondensationsgrad vorliegt, dem Produkt (P) zum Abbruch der Reaktion ein Produkt mit gegenüber der fokalen Gruppe von (P) reaktiven Gruppen zugesetzt werden. So kann bei einer Carbonatgruppe bzw. Carbamoylgruppe als fokaler Gruppe zum Beispiel ein Mono-, Di- oder Polyamin zugegeben werden. Bei einer Hydroxylgruppe als fokaler Gruppe kann dem Produkt (P) beispielsweise ein Mono-, Di- oder Polyisocyanat, eine Epoxydgruppen enthaltende Verbindung oder ein mit OH-Gruppen reaktives Säurederivat zugegeben werden.

Durch die vorgenannte Einstellung der Reaktionsbedingungen und gegebenenfalls durch die Wahl des geeigneten Lösemittels können die erfindungsgemäßen Produkte nach der Herstellung ohne weitere Reinigung weiterverarbeitet werden. Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bei Temperaturen von beispielsweise 10 bis 100 °C, bevorzugt 20 bis 80 °C und besonders bevorzugt 30 bis 60 °C unterworfen werden. Gegebenenfalls kann das Reaktionsgemisch auch zur Entfernung von eventuell vorhandenen Ausfällungen filtriert werden. In einer weiteren bevorzugten Ausführungsform wird das Produkt gestrippt, das heißt von niedermolekularen, flüchtigen Verbindungen befreit. Dazu kann nach Erreichen des gewünschten Umsatzgrades der Katalysator optional desaktiviert und die niedermolekularen flüchtigen Bestandteile, zum Beispiel Monoalkohole, Phenole, Carbonate, Chlorwasserstoff oder leichtflüchtige oligomere oder cyclische Verbindungen destillativ, gegebenenfalls unter Einleitung eines Gases, vorzugsweise Stickstoff, Kohlendioxid oder Luft, gegebenenfalls bei vermindertem Druck, entfernt werden.

Die hyperverzweigten Polycarbonate, die wie vorstehend beschrieben erhältlich sind, weisen in der Regel eine Glasübergangstemperatur von weniger als 50 °C, bevorzugt weniger als 30 und besonders bevorzugt weniger als 10 °C auf. Die OH-Zahl beträgt meist mindestens 30 mg KOH/g , bevorzugt zwischen 50 und 250 mg/g. Das gewichtsmittlere Molgewicht M_{w} liegt zumeist zwischen 1.000 und 150.000, bevorzugt von 1500 bis 100.000 g/mol, das zahlenmittlere Molgewicht Mₙ zwischen 500 und 50.000, bevorzugt zwischen 1.000 und 40.000 g/mol. Das hyperververzweigte Polycarbonat ist meist nicht löslich oder dispergierbar in Wasser, das heißt, dass es nicht möglich ist eine klare (d.h. ohne Partikel, die mit dem blossen Auge erkennbar sind) wässrige Lösung oder Dispersion herzustellen.

Das Amphiphil enthält bevorzugt ein hyperverzweigtes Polycarbonat, das verknüpft ist mit mindestens einem linearen oder Kamm-förmigen Polymer und/oder mit mindestens einer funktionalen C₁-C₂₄-Einheit enthaltend eine Säuregruppe, eine Aminogruppe oder mindestens zwei Hydroxygruppen. Besonders bevorzugt enthält das Amphiphil ein hyperverzweigtes Polycarbonat, das verknüpft ist mit mindestens einem linearen oder Kamm-förmigen Polymer oder mit mindestens einer funktionalen C₁-C₂₄-Einheit enthaltend eine Säuregruppe, eine Aminogruppe oder mindestens zwei Hydroxygruppen. Ganz besonders bevorzugt enthält das Amphiphil ein hyperverzweigtes Polycarbonat, das verknüpft ist mit mindestens einem linearen oder Kamm-förmigen Polymer. Das Molverhältnis von hyperverzweigtem Polycarbonat zur Summe aus linearem oder Kamm-förmigem Polymer und aus funktionaler C₁-C₂₄-Einheit liegt meist im Bereich von 1 : 1 bis 1 : 100, bevorzugt 1 : 1 bis 1 : 50, besonders bevorzugt 1 : 1 bis 1 : 25.

Die erfindungsgemäße Zusammensetzung enthält meist 0,01 bis 40 Gew.%, bevorzugt 0,05 bis 30 Gew.%, besonders bevorzugt 0,1 bis 20 Gew.% Amphiphil. Das Amphiphil ist meist löslich oder dispergierbar in Wasser, das heißt, dass es möglich ist eine klare (d.h. ohne Partikel, die mit dem blossen Auge erkennbar sind) wässrige Lösung oder Dispersion herzustellen.

Das Amphiphil enthält in einer bevorzugten Ausführungsform ein hyperverzweigtes Polycarbonat, das verknüpft ist mit mindestens einem linearen oder Kamm-förmigen Polymer. Die Verknüpfung erfolgt üblicherweise mittels eines Linkers.

Bevorzugt ist das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis mindestens eines polaren, ethylenisch ungesättigten Monomers, oder
c) ein Polykondensat enthaltend Polyethylenglykol, oder
d) ein Polyethylenglykol,
wobei das Polyethylenglykol d) über einen Linker mit dem Polycarbonat vernüpft ist. Besonders bevorzugt ist das lineare Polymer eines der vorgenannten Polymere a), b) oder c). In einer weiteren besonders bevorzugten Ausführungsform ist das lineare Polymer eines der vorgenannten Polymere a), c) oder d). Speziell bevorzugt ist das lineare Polymer eines der vorgenannten Polymere a) oder c), insbesondere a).

Das lineare Polymer kann in einer Ausführungsform ein Homopolymer oder statistisches Copolymer enthaltend ein polares, ethylenisch ungesättigtes Monomer sein. Die zahlenmittlere Molmasse Mn liegt meist unter 100000 g/mol, bevorzugt unter 50.000 g/mol, besonders bevorzugt unter 20.000 g/mol und ganz besonders bevorzugt unter 10.000 g/mol und kann durch GPC und einem geeigneten Standard ermittelt werden. Mn liegt üblicherweise über 200 g/mol, bevorzugt über 500 g/mol.

Geeignete polare, ethylenisch ungesättigte Monomere sind Ladung tragende oder ionisierbare Gruppen tragende Monomere, die eine polymerisierbare, ethylenisch ungesättige Bindung enthalten. Beispiele für Ladung tragende oder ionisierbare Gruppen sind Carbonsäure, Sulfonsäure, Polyethylenglykol, Alkohol, Nitril, Amid, Amin, Dialkylamin. Beispiele für polare, ethylenisch ungesättigte Monomere sind Vinylpyrrolidon, (Meth)Acrylsäure, ein Sulfonsäure-haltiges (Meth)Acrylat (wie Acrylamido-2-methyl-propansulfonsäure), ein Amino-funktionelles (Meth)Acrylat (wie Dimethylaminoethyl(meth)acrylat), (Meth)Acrylsäureester von einem Polyethylenglykolderivat (wie Polyethylenglykolmonomethylether(meth)acrylat), Itaconsäure, Maleinsäureanhydrid, OH-Gruppen substituierte C₁-C₂₀-Alkyl(meth)acrylate (wie Hydroxylethyl(meth)acrylat, Hydroxybutyl(meth)acrylat), (Meth)Acrylnitril, (Meth)Acrylamid, N-Methylol(meth)-acrylamid. Bevorzugte polare, ethylenisch ungesättigte Monomere sind Vinylpyrrolidon, (Meth)Acrylsäure, Polyethylenglykolmonomethylether(meth)acrylat, Polyethylenglykol(meth)acrylat. Der Ausdruck "(Meth)Acryl" bedeutet "Acryl" oder "Methacryl".

Beispiele für lineare Homopolymere enthaltend ein polares, ethylenisch ungesättigtes Monomer sind Homopolymere der vorgenannten polaren, ethylenisch ungesättigten Monomere, bevorzugt von Vinylpyrrolidon, (Meth)Acrylsäure, Polyethylenglykolmono-methylether(meth)acrylat, Polyethylenglykol(meth)acrylat.

Beispiele für statistische Copolymere enthaltend ein polares, ethylenisch ungesättigtes Monomer sind Copolymere der vorgenannten polaren, ethylenisch ungesättigten Monomere, bevorzugt von Vinylpyrrolidon, (Meth)Acrylsäure, Polyethylenglykolmonomethylether(meth)acrylat, Polyethylenglykol(meth)acrylat. Als weiteres Monomer kann das statistische Copolymer enthalten: Ester der Acrylsäure mit C1-C10-Alkanolen wie Ethylacrylat, n-Butylacrylat, Isobutylacry-lat, tert.-Butylacrylat, n-Hexylacrylat, 2-Ethylhexylacrylat und 3-Propylheptylacrylat, die Ester der Methacrylsäure mit C1-C10-Alkanolen wie Methylmethacrylat, Ethylmethacrylat, n-Butylmethacrylat, Isobutylmethacrylat, tert.-Butylmethacrylat und n-Hexylmethacrylat, N-(C2-C10-Alkyl)amide der Acrylsäure und der Methacrylsäure sowie die N-(C1-C2-Alkyl)-N-(C2-C10-alkyl)amide der Acrylsäure und der Methacrylsäure, z. B. N-Ethylacrylamid, N,N-Diethylacrylamid, N-Butylacrylamid, N-Methyl-N-propylacrylamid, N-(n-Hexyl)acrylamid, N-(n-Octyl)-acrylamid und die entsprechenden Methacrylamide, vinylaromatische Monomere wie Styrol, Methylstryrol, Vinyltoluol, Olefine mit 2 bis 10 C-Atomen, vorzugsweise α-Olefine mit 3 bis 10 C-Atomen wie Propen, 1-Buten, 1-Penten, 1-Hexen, 1-Octen und 1-Decen, Vinylester ali¬phatischer Carbonsäuren wie Vinylacetat, Vinylpropionat, Vinyllaurat, Vinylnonanoat, Vinyldecanoat, Vinyllaurat und Vinylstearat, ungesättigte Nitrile wie Acrylnitril und Methacrylnitril, halogenierte Olefine wie Vinylchlorid, C11-C20-Alkylester monoethylenisch ungesättigter Monocarbonsäuren mit vorzugsweise 3 bis 6 C-Atomen, z. B. C11-C20-Alkylacrylate und C11-C20-Alkylmethacrylate wie Laurylacrylat, Laurylmethacry-lat, Isotridecylacrylat, Isotridecylmethacrylat, Stearylacrylat, Stearylmethacrylat, Di-C1-C20-Alkylester ethylenisch ungesättigter Dicarbonsäuren mit vorzugsweise 4 bis 8 C-Atomen, z. B. Di-C1-C20-Alkylester der Fumarsäure und der Maleinsäure wie Di-methylfumarat, Dimethylmaleat, Dibutylfumarat und Dibutylmaleat, Glycidylester mo-noethylenisch ungesättigter Monocarbonsäuren mit vorzugsweise 3 bis 6 C-Atomen, wie Glycidylacrylat und Glycidylmethacrylat. Bevorzugte weitere Monomere sind die Ester mit C1-C10-Alkanolen der Acrylsäure und der Methacrylsäure.

Das lineare Polymer kann in einer weiteren Ausführungsform ein Blockpolymer enthaltend einen Block aus Polyethylenglykol oder aus mindestens einem polaren, ethylenisch ungesättigten Monomer sein. Die Molmasse Mn liegt meist im Bereich von 200-10.000 g/mol, bevorzugt zwischen 300 und 2000 g/mol, und kann durch GPC ermittelt werden. Das Blockpolymer kann vom A-B oder A-B-A, bevorzugt A-B Typ sein. Die Herstellung von Blockpolymeren dieser Typen ist allgemein bekannt. Geeignete und bevorzugte polare, ethylenisch ungesättigte Monomere sind wie oben genannt. Beispiele für einen Block aus Polyethylenglykol sind Polyethylenglykol oder Polyethylenglykolmonoalkylether mit einer Molmasse Mn von 200 bis 10000 g/mol. Beispiele für einen Block aus mindestens einem polaren, ethylenisch ungesättigten Monomer sind Polyvinylpyrrolidon oder Poly(meth)acrylsäure oder Polyethylenglykolmonomethylether(meth)acrylat. Der jeweils andere Block kann aus Polymerblöcken aus dem Stand der Technik aufgebaut sein. Bevorzugt ist der andere Block unpolar, beispielsweise ist er aus Caprolacton oder Propylenoxid aufgebaut. In einer weiteren Ausführungsform enthält der andere Block Polyester (z.B. basierend auf einer Dicarbonsäure und einem Diol), Polyamid (z.B. basierend auf einer Dicarbonsäure und einem Diamin), Polycarbonat, Polyurethan oder Polyharnstoff. Bevorzugte Blockpolymere sind Polyethylengly-kol-block-polycaprolacton und Polyethylenglykolmonomethylether-blockpolycaprolacton und Polyproyplenglykol-block-Polyethylenglykol.

Das lineare Polymer kann in einer weiteren Ausführungsform ein Polykondensat enthaltend Polyethylenglykol sein. Der Begriff Polykondensat schließt im Sinne der vorliegenden Erfindung auch Polyadditionsprodukte ein. Beispiele für Polyethylenglykol sind Polyethylenglykol oder Polyethylenglykolmonoalkylether mit einer Molmasse Mn von 200 bis 10000 g/mol. Beispiele für Polykondensate sind Polyether, Polyamide, Polyimide, Polyester, Polycarbonate, Polyurethane und Polyharnstoffe, bevorzugt Polyether und Polyester. Bevorzugtes Polykondensat ist ein Polyether basierend auf C₃-C₂₄ Alkylenoxid, besonders Propylenoxid, und ein Polyester auf Basis von Hydroxycarbonsäureverbindungen, Dialkoholverbindungen oder Disäureverbindungen, besonders Hydroxycarbonsäureverbindungen. Bevorzugte Hydroxycarbonsäureverbindungen sind Lactone, insbesondere C₄ bis C₁₈-Alkyllactone, ganz besonders bevorzugt ε-Capro-lacton.

Das lineare Polymer kann in einer weiteren Ausführungsform ein Polyethylenglykol sein, wobei das Polyethylenglykol über einen Linker mit dem Polycarbonat vernüpft ist. Bevorzugt ist der Linker ein Polyisocyanat. Beispiele für Polyethylenglykol sind Polyethylenglykol oder Polyethylenglykolmonoalkylether mit einer Molmasse Mn von 200 bis 10000 g/mol, bevorzugt 300-2000 g/mol. Bevorzugt handelt es sich bei dem Polyethylenglykol um ein Polyethylenglykolmono-C₁-C₁₈-alkylether, insbesondere um ein ein Polyethylenglykolmonomethylether.

Unter Kamm-förmigen Polymeren werden hier Kammpolymere verstanden, die üblicherweise an einer linearen Hauptkette in mehr oder weniger regelmäßigen Abständen längere, untereinander nahezu gleichlange Seitenketten, bevorzugt aliphatische Seitenketten, enthalten. Die Molmasse Mn liegt meist im Bereich von 500 bis 100000 g/mol und kann durch GPC ermittelt werden. Das Kamm-förmige Polymer enthält bevorzugt Polyalkylenglykol-mono(meth)acrylat oder Allylalkoholalkoxylat (wie Polyethylenglykol-allylether,) in polymerisierter Form, bevorzugt Polyethylenglykolmonoalkylether(meth)acrylat mit einer Molmasse Mn von 100 bis 5000 g/mol. Besonders bevorzugt enthält das Kammpolymer Polyethylenglykolmonomethylether-acrylat oder Polyethylenglykolmonomethylether-methacrylat mit einer Molmasse Mn von jeweils 100 bis 3000 g/mol, bevorzugt 200 bis 1500 g/mol. Das Kammpolymer kann zusätzlich zu Polyalkylenglykol-mono(meth)acrylat oder Allylalkoholalkoxylate beliebige copolymerisierbare ethylenisch ungesättigte Monomere enthalten. Bevorzugte zusätzliche Monomere sind unpolare Monomere und/oder vorgenannte polare, ethylenisch ungesättigte Monomere. Bevorzugte unpolare Monomere sind C₁-C₂₀-Alkyl(meth)acrylate oder Vinylaromaten mit bis zu 20 C-Atomen. Beispiele umfassen Methyl(meth)acrylat, Ethyl(meth)acrylat, n-Butyl(meth)acrylat, 2-Ethylhexyl(meth)acrylat, Lauryl(meth)acrylat oder 4-t-Butyl-cyclohexyl(meth)acrylat. Als vinylaromatische Verbindungen kommen z.B. Vinyltoluol, α-Butylstyrol, 4-n-Butylstyrol, 4-n-Decylstyrol oder Styrol in Betracht. Bevorzugte zusätzliche Monomere sind Methy(meth)acrylat, Laurylacrylat, Stearylacrylat, Styrol, Vinylpyrrolidon oder deren Mischungen.

Das lineare oder Kamm-förmige Polymer kann nach allgemein bekannten Verfahren hergestellt werden (beispielsweise aus US 5,556,918 und EP-A 742 238). In einer Ausführungsform wird das lineare Polymer, das ein Homopolymer oder statistisches Copolymer ist enthaltend ein polares, ethylenisch ungesättigtes Monomer, das Blockpolymer enthaltend einen Block aus Polyethylenglykol oder auf Basis mindestens eines polaren, ethylenisch ungesättigten Monomer, sowie das Kammpolymer durch radikalisch initiierte Lösungspolymerisation der Monomere in Gegenwart eines Initiators und gegebenenfalls eines Reglers hergestellt. Bevorzugt wird dabei ein Initiator verwendet, der beim Zerfall ein Hydroxyl-Radikal (OH-Radikal) bildet und/oder ein Regler der eine OH-Gruppe oder eine NH₂-Gruppe enthält. Diese OH- oder NH₂-Gruppen könne später als Linker-reaktive Gruppe verwendet werden.

Geeignete Initiatoren sind organische Hydroperoxide wie tert.-Butylhydroperoxid, Tetrahydrofuranhydroperoxid, Cumolhydroperoxid oder 2,2'-Azobis(2-methyl-N-(2-hydroxyethyl)propionamid). Geeignete Regler sind Aminoalkohole, Aminophenole und insbesondere Thioalkanole wie 3-Hydroxypropanthiol, 3-Mercapto-1,2-propandiol, 2-Hydroxyethyl-3-mercaptopropionsäureester und vor allem 2-Hydroxyethanthiol (Mercaptoethanol). Sofern ein derartiger Regler eingesetzt wird, kann die Polymerisation auch in Gegenwart eines konventionellen Initiators durchgeführt werden, beispielsweise eines konventionellen Azostarters oder eines organischen Peroxids wie Azobis-(isobutyronitril), Di-(tert.-butyl)peroxid, Didecanoylperoxid, Dibenzoylperoxid, Peressigsäure-tert.-butylester oder 2-Methylperpropionsäure-tert.-butylester. Sofern man die Polymerisation in Gegenwart eines der vorgenannten Regler durchführt, wird man den Regler in der Regel in einer Menge von 0,1 bis 12 Gew.-%, häufig 0,2 bis 8 Gew.-% und insbesondere 0,5 bis 5 Gew.-%, bezogen auf die Gesamtmenge der Monomere einsetzen. Initiatoren werden in der Regel in einer Menge von 0,05 bis 5 Gew.-%, häufig 0,1 bis 4 Gew.-% und besonders bevorzugt in einer Menge von 0,2 bis 3 Gew.-%, bezogen auf die zu polymerisierenden Monomere eingesetzt. Wegen weiterer Details wird insbesondere auf die Seite 3 der EP 742 238 verwiesen, auf deren Offenbarung Bezug genommen wird.

Das hyperverzweigte Polycarbonat, das verknüpft ist mit der funktionalen C₁-C₂₄-Einheit, ist Üblicherweise erhältlich, bevorzugt wird es erhalten, durch Umsetzung des hyperverzweigten Polycarbonats mit einem Funktionalisierungsreagenz, das die funktionale C₁-C₂₄-Einheit enthaltend eine Säuregruppe, eine Aminogruppe oder mindestens zwei Hydroxygruppen und die Verküpfungsgruppe umfasst, und optional mit einem Linker.

Geeignete Funktionalisierungsreagenzien zur direkten kovalenten Verknüpfung ohne Linker sind zyklische Carbonsäureanhydride, wie Bernsteinsäureanhydrid oder Phthalsäureanhydrid, insbesondere Bemsteinsäureanhydrid. Typischerweise werden die Anhydride mit dem hyperverzweigten Polycarbonat unter erhöhten Temperaturen, meist bei 80 bis 200 °C, umgesetzt. Die Umsetzung kann mit oder ohne Zusatz von Lösungsmitteln erfolgen. Eine weitere Aufreinigung ist normalerweise nicht nötig.

Geeignete Funktionalisierungsreagenzien zur kovalenten Verknüpfung mittels Linker sind Hydroxycarbonsäuren, Aminocarbonsäuren, Hydroxysulfonsäuren, Hydroxysulfate, Aminosulfonsäuren oder Aminosulfate, Hydroxyamine (wie Diethanolamin), Polyamine (z.B. Diethylentetramin), oder Polyole (z.B. Glycerin, Trimethylolpropan, Pentaerythrit). Bevorzugte Linker hierfür sind nachstehend beschriebene Polyisocyanate, bevorzugt Diisocyanate, besonders bevorzugt aliphatische Diisocyanate (wie Hexamethylendiisocyanat und Isophorondiisocyanat).

Die Verknüpfung der linearen oder Kamm-förmigen Polymere mit dem hyperverzweigten Polycarbonat gelingt bevorzugt mit Hilfe eines Linkers. Meist wird dabei zunächst der Linker mit dem linearen oder Kamm-förmigen Polymer kovalent verbunden, um anschließend das Linker-haltige Polymer an das hyperverzweigte Polycarbonat anzukuppeln. Damit das Linker-haltige Polymer hergestellt werden kann, enthält das Ausgangspolymer meist eine Gruppe, die mit dem Linker reagieren kann (Linker-reaktive Gruppe). Die mittlere Anzahl der Linker-reaktive Gruppen beträgt in der Regel nicht mehr als zwei und liegt vorzugsweise im Bereich von 0,3 bis 1,8, insbesondere im Bereich von 0,5 bis 1,5 und speziell im Bereich von 0,6 bis 1,4 pro Polymermolekül. Die Linker-reaktive Gruppe kann in der Polymerkette angeordnet sein oder befindet sich vorzugsweise am Ende der Polymerkette.

Im Falle eines linearen Polymers, das ein Homopolymer oder statistisches Copolymer ist enthaltend ein polares, ethylenisch ungesättigtes Monomer, ein Blockpolymer enthaltend einen Block aus Polyethylenglykol oder auf Basis mindestens eines polaren, ethylenisch ungesättigten Monomers, oder eines Kammpolymers kann die Linker-reaktive Gruppe wie vorbeschrieben über einen geeigneten Initiator und/oder Regler eingeführt werden. Alternativ kann die Linker-reaktive Gruppe über eine kontrollierte radikalische Reaktion nach dem Stand der Technik (wie Atom Transfer Radical Polymerization (ATRP), Reversible Addition Fragmentation Chain Transfer Polymerization (RAFT), oder Nitroxide Mediated Polymerization (NMP)) gezielt am Kettenende eingeführt werden. Ebenso ist es möglich, dass eine funktionelle Gruppe in der Polymerkette als Linker-reaktive Gruppe verwendet wird, beispielsweise eine von gegebenenfalls mehreren OH-Gruppen eines einpolymerisierten Hydroxyethyl(meth)acrylats.

Im Falle eines Polykondensats enthaltend Polyethylenglykol kann eine Linker-reaktive Gruppe am Kettenende des Polykondensats erzeugt werden durch eine geeignete Stöchiometrie und Einsatz eines monofunktionellen Monomers. Bevorzugt wird die Linker-reaktive Gruppe durch ring-öffnende Polymerisation eines Lactons erzeugt, so dass genau eine funktionelle Hydroxygruppe am Kettenende entsteht.

Im Falle eines Polyethylenglykol kann als Linker-reaktive Gruppe eine Hydroxy-Gruppe am Kettenende verwendet werden. Bevorzugt werden Polyethylenglykolmonoalkylether, die genau eine Linker-reaktive Gruppe am Kettenende haben.

Allgemein kommen als Linker Polyisocyanate in Betracht mit einer Funktionalität bezüglich der Isocyanatgruppen von wenigstens 1,5, insbesondere 1,5 bis 4,5 und speziell 1,8 bis 3,5 umfassen aliphatische, cycloaliphatische und aromatische Di- und Polyisocyanate sowie die Isocyanurate, Allophanate, Urethdione und Biurete von aliphatischen, cycloaliphatischen und aromatischen Diisocyanaten. Vorzugsweise weisen die Polyisocyanate im Mittel 1,8 bis 3,5 Isocyanatgruppen pro Molekül auf. Beispiele für geeignete Polyisocyanate sind aromatische Diisocyanate wie Toluol-2,4-diisocyanat, Toluol-2,6-diisocyanat, kommerziell erhältliche Mischungen von Tolu-ol-2,4- und -2,6-diisocyanat (TDI), n-Phenylendiisocyanat, 3,3'-Diphenyl-4,4'-biphenylendiisocyanat, 4,4'-Biphenylendiisocyanat, 4,4'-Diphenylmethandiisocyanat, 2,4'-Diphenylmethandiisocyanat, 3,3'-Dichlor-4,4'-biphenylendiisocyanat, Cumen-2,4-diisocyanat, 1,5-Naphthalindiisocyanat, p-Xylylendiisocyanat, p-Phenylendiisocyanat, 4-Methoxy-1,3-phenylendiisocyanat, 4-Chlor-1,3-phenylendiisocyanat, 4-Ethoxy-1,3-phenylendiisocyanat, 2,4-Dimethylen-1,3-phenylendiisocyanat, 5,6-Dimethyl-1,3-phenylendiisocyanat, 2,4-Diisocyanatodiphenylether, aliphatische Diisocyanate wie Ethylendiisocyanat, Ethylidendiisocyanat, Propylen-1,2-diisocyanat, 1,6-Hexamethylendiisocyanat (HDI), 1,4-Tetramethylendiisocyanat, 1,10-Decamethylen-diisocyanat und cycloaliphatische Diisocyanate, wie Isophorondiisocyanat (IPDI), Cyclohexylen-1,2-diisocyanat, Cyclohe-xylen-1,4-diisocyanat und Bis(4,4'-Isocyanatocyclohexyl)methan. Unter den Polyisocyanaten sind solche bevorzugt, deren Isocyanatgruppen sich in ihrer Reaktivität unterscheiden, wie Toluol-2,4-diisocyanat, Toluol-2,6-diisocyanat, 4'-Diphenylmethandiisocyanat, cis- und trans-Isophorondiisocyanat, oder Mischungen dieser Verbindungen.

Die Umsetzung mit dem Polyisocyanat erfolgt in Schmelze oder in einem organischen Lösungsmittel, bevorzugt in einem aprotisch-polaren organischen Lösungsmitteln oder Gemischen solcher Lösungsmittel. Beispiele sind Ketone (beispielsweise Aceton), Butylacetat, Tetrahydrofuran (THF), Xylol, Chlorbenzol, Dimethylsulfoxid (DMSO) oder Dimethylformamid (DMF). Bevorzugte Lösungsmittel sind Butylacetat, Xylol und Aceton. Die Umsetzung erfolgt üblicherweise bei erhöhten Temperaturen, wobei sich die-Temperatur auch nach der Siedetemperatur des gewählten Lösungsmittels richtet. Die Umsetzung des Polyisocyanats mit der ersten Komponente kann bei 20 bis 80 °C, gewünschtenfalls aber auch bis 100 °C erfolgen. Die Umsetzung der weiteren Isocyanatgruppe kann bei Temperaturen von 50 bis 100 °C erfolgen.

Die Umsetzung kann äquimolar erfolgen, was bedeutet, dass das Mengenverhältnis so gewählt wird, dass pro Mol umzusetzender Hydroxyl-Gruppe des Funktionalisierungsreagenz oder des linearen oder des kammartigen Polymers 1 Mol Diisocyanat eingesetzt wird. Bevorzugt wird mit leichtem (z.B. 0 bis 15 mol%) Überschuss der HydroxylGruppen gearbeitet, um die Menge an nicht umgesetztem Diisocyanat zu verringern. Ist das radikalische Copolymer über einen Initiator oder Regler OH-funktionalisiert, wird das Diisocyanat äquimolar oder im leichten Unterschuss zu den darüber eingeführten OH Gruppen umgesetzt. Im Falle symmetrischer Diisocyanate (wie HDI) kann es sich auch empfehlen, einen Überschuss an Diisocyanat einzusetzen und den Überschuss anschliessend destillativ zu entfernen.

Vorzugsweise wird die Umsetzung in Gegenwart eines Katalysators durchgeführt. Als Katalysatoren eignen sich beispielsweise tertiäre Amine, z. B. Triethylamin, Tri-n-propylamin, N-Methylpyrrolidin, N-Methylpiperidin und Diazabicyclooctan (DABCO), Zink-Carboxylate, Wismut-Carboxylate, Titan-Alkoholate, zinnorganische Verbindungen, insbesondere Dialkylzinn(IV)salze von aliphatischen Carbonsäuren wie Dibutylzinndilaurat und Dibutylzinndioctoat, Zinn(II)dialkanoate wie Zinndioctoat, sowie Cäsiumsalze wie Cäsiumacetat. In einer Ausführungsform sind Zink-Carboxylate, Wismut-Carboxylate, Titan-Alkoholate besonders geeignet, wobei die Carboxylate bevorzugt C₁-C₂₀ Carboxylate (wie Formiat, Acetat, Propionat, Hexanoat, Octanoat oder Neodecanoat) sind. Der Katalysator kann in Mengen von 50 bis 50000 ppm, vorzugsweise 100 bis 5000 ppm, bezogen auf gesamten Feststoff, eingesetzt werden.

Die Umsetzung wird üblicherweise bei erhöhten Temperaturen im Bereich von 40 bis 120 °C durchgeführt. Welche Temperatur im Einzelnen gewählt wird, hängt von der Art des verwendeten organischen Lösungsmittels ab. Das Lösungsmittel kann anschliessend durch Destillation entfernt werden.

Üblicherweise wird man die Umsetzung so durchführen, dass zunächst die Komponente, die Isocyanatgruppen-funktionalisiert werden soll (beispielweise das lineare oder Kamm-förmige Polymer oder die funktionale C₁-C₂₄-Einheit), mit dem Diisocyanat in Gegenwart des Katalysators und eines Lösungsmittels solange umgesetzt wird, bis der Isocyanat-Wert im Reaktionsgemisch auf die Hälfte gesunken ist. Bei Verwendung eines leichten Hydroxygruppen-Überschusses wird solange umgesetzt, bis der theoretische Endwert dem vollständigen Umsatz der Hydroxygruppen-Gruppen entspricht. Dies lässt sich auf bekannte Weise beispielsweise titrimetrisch ermitteln. Danach erfolgt dann die Zugabe der anderen Komponente (beispielsweise hyperverzweigtes Polycarbonat). Das molare Verhältnis von hyperverzweigtem Polycarbonat zu linearem Polymer, Kamm-förmigem Polymer oder zur funktionalen C₁-C₂₄-Einheit liegt dabei bei 1 : 1 bis 1 : 25, bevorzugt bei 1 : 2 bis 1 : 15. Die Reaktion wird fortgeführt, bis der Isocyanatwert auf Null gesunken ist.

Die erfindungsgemäße Zusammensetzung ist erhältlich indem man das Amphiphil und den Wirkstoff, der in Wasser bei 20 °C zu höchstens 10 g/L löslich ist, in Kontakt bringt, wobei das Amphiphil ein hyperverzweigtes Polycarbonat enthält, das verknüpft ist mit mindestens einem linearen oder Kamm-förmigen Polymer oder mindestens einer funktionalen C₁-C₂₄-Einheit enthaltend eine Säuregruppe, eine Aminogruppe oder mindestens zwei Hydroxygruppen. Die Komponenten können durch allgemein bekannte Methoden, wie Mischen, Emulgieren oder Suspendieren, in Kontakt gebracht werden.

Das Gewichtsverhältnis von Wirkstoff zu Amphiphil liegt meist im Bereich von 100 : 1 bis 1 : 100, bevorzugt 10 : 1 bis 1 : 50, besonders bevorzugt 2 : 1 bis 1 : 25. Der Wirkstoff kann in gelöster Form oder in fester, partikulärer Form vorliegen. Die Wirkstoffpartikel können kristallin oder amorph sein. Die Partikelgröße kann 1 nm bis 10 µm betragen. Die Zusammensetzung kann eine Lösung, Emulsion, Suspension oder Suspoemulsion des Wirkstoffs sein. Die erfindungsgemäße Zusammensetzung ist bevorzugt eine wässrige Zusammensetzung. Bevorzugt enthält sie mindestens 40 Gew.%, besonders bevorzugt mindestens 60 Gew.%, und insbesondere mindestens 80 Gew.% Wasser. Üblicherweise enthält die Zusammensetzung höchstens 99 Gew.% Wasser.

Die erfindungsgemäße Zusammensetzung kann Formulierungshilfsmittel enthalten, wobei sich die Wahl der Hilfsmittel üblicherweise nach der konkreten Anwendungsform bzw. dem Wirkstoff richtet. Beispiele für geeignete Formulierungshilfsmittel sind Lösungsmittel, feste Trägerstoffe, oberflächenaktive Stoffe (wie Tenside, Schutzkolloide, Netzmittel und Haftmittel), organische und anorganische Verdicker, Bakterizide, Frostschutzmittel, Entschäumer, ggf. Farbstoffe und Kleber (z. B. für Saatgutbehandlung).

Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z. B. von Lignin-(Borresperse^{®}-Typen, Borregaard, Norwegen), Phenol-, Naphthalin-(Morwet^{®}-Typen, Akzo Nobel, USA) und Dibutylnaphthalinsulfonsäure (Nekal^{®}-Typen, BASF, Deutschland), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol^{®}-Typen, Clariant, Schweiz), Polycarboxylate (Sokalan^{®}-Typen, BASF, Deutschland), Polyalkoxylate, Polyvinylamin (Lupamin^{®}-Typen, BASF, Deutschland), Polyethylenimin (Lupasol^{®}-Typen, BASF, Deutschland), Polyvinylpyrrolidon und deren Copolymere in Betracht.

Als Tenside kommen insbesondere anionische, kationische, nicht-ionische und amphotere Tenside, Blockpolymere und Polyelektrolyte in Betracht. Geeignete anionische Tenside sind Alkali-, Erdalkali- oder Ammoniumsalze von Sulfonaten, Sulfaten, Phosphaten oder Carboxylaten. Beispiele für Sulfonate sind Alkylarylsulfonate, Diphenylsulfonate, alpha-Olefinsulfonate, Sulfonate von Fettsäuren und Ölen, Sulfonate von ethoxylierten Alkylphenolen, Sulfonate von kondensierten Naphthalinen, Sulfonate von Dodecyl und Tridecylbenzolen, Sulfonate von Naphthalinen und Alkylnaphthalinen, Sulfosuccinate oder Sulfosuccinamate. Beispiele für Sulfate sind Sulfate von Fettsäuren und Ölen, von ethoxylierten Alkylphenolen, von Alkoholen, von ethoxylierten Alkoholen, oder von Fettsäureestern. Beispiele für Phosphate sind Phosphatester. Beispiele für Carboxylate sind Alkylcarboxylate und carboxylierte Alkohol- oder Alkylphenolethoxylate.

Geeignete nicht-ionische Tenside sind Alkoxylate, N-alkylierte Fettsäureamide, Aminoxide, Ester oder Zucker-basierte Tenside. Beispiele für Alkoxylate sind Verbindungen, wie Alkohole, Alkylphenole, Amine, Amide, Arylphenole, Fettsäuren oder Fettsäureester, die alkoxyliert wurden. Zur Alkoxylierung kann Ethylenoxid und/oder Propylenoxid eingesetzt werden, bevorzugt Ethylenoxid. Beispiele für N-alkylierte Fettsäureamide sind Fettsäureglucamide oder Fettsäurealkanolamide. Beispiele für Ester sind Fettsäureester, Glycerinester oder Monoglyceride. Beispiele für Zucker-basierte Tenside sind Sorbitane, ethoxilierte Sorbitane, Saccharose- und Glucoseester oder Alkylpolyglucoside. Geeignete kationische Tenside sind quarternäre Tenside, beispielsweise quartäre Ammonium-Verbindungen mit einer oder zwei hydrophoben Gruppen, oder Salze langkettiger primärer Amine. Geeignete amphothere Tenside sind Alkylbetaine und Imidazoline. Geeignete Blockpolymere sind Blockpolymere vom A-B oder A-B-A Typ umfassend Blöcke aus Polyethylenoxid und Polypropylenoxid oder vom A-B-C Typ umfassend Alkanol, Polyethylenoxid und Polypropylenoxid. Geeignete Polyelektrolyte sind Polysäuren oder Polybasen. Beispiele für Polysäuren sind Alkalisalze von Polyacrylsäure. Beispiele für Polybasen sind Polyvinylamine oder Polyethylenamine.

Die erfindungsgemäße Zusammensetzung kann hohe Mengen oberflächenaktiver Stoffe und Tensid umfassen. Sie kann 0,1 bis 40 Gew.%, bevorzugt 1 bis 30 und insbesondere 2 bis 20 Gew.% Gesamtmenge von oberflächenaktiven Stoffe und Tensiden umfassen bezogen auf die Gesamtmenge der Zusammensetzung.

Beispiele für Adjuvantien sind organisch modifizierte Polysiloxane, wie BreakThruS 240^{®}; Alkoholalkoxylate, wie Atplus^{®}245, Atplus^{®}MBA 1303, Plurafac^{®}LF und Lutensol^{®} ON ; EO-PO-Blockpolymerisate, z. B. Pluronic^{®} RPE 2035 und Genapol^{®} B; Alkoholethoxylate, z. B. Lutensol^{®} XP 80; und Natriumdioctylsulfosuccinat, z. B. Leophen^{®} RA. Beispiele für Verdicker (d. h. Verbindungen, die der Zusammensetzung ein modifiziertes Fließverhalten verleihen, d. h. hohe Viskosität im Ruhezustand und niedrige Viskosität im bewegten Zustand) sind Polysaccharide sowie organische und anorganische Schichtmineralien wie Xanthan Gum (Kelzan^{®}, CP Kelco), Rhodopol^{®} 23 (Rhodia) oder Veegum^{®} (R.T. Vanderbilt) oder Attaclay^{®} (Engelhard Corp.).

In einer bevorzugten Ausführungsform ist der Wirkstoff ein Pestizid und die erfindungsgemäßen Zusammensetzungen liegen in Form einer agrochemischen Formulierung vor. Geeignete agrochemische Formulierungen sind wasserlösliche Konzentrate (SL, LS), redispergierbare Konzentrate (DC), emulgierbare Konzentrate (EC), Emulsionen (EW, EO, ES, ME), Suspensionen (SC, OD, FS) oder Suspoemulsionen (SE). Bevorzugt liegt die Zusammensetzung vor in Form eines emulgierbaren Konzentrates (EC), eines Suspensionskonzentrats (SC), eines wasserlöslichen Konzentrats (SL), einer Lösung für Saatgutbehandlung (LS), oder eines redispergierbaren Konzentrats (DC).

Die agrochemischer Formulierung wird meist vor der Anwendung verdünnt um den sogenannten Tankmix herzustellen. Zur Verdünnung kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht. Bevorzugt wird Wasser verwendet. Es ist auch möglich, das Amphiphil erst dem Tankmix zuzusetzen. In dieser Ausführungsform liegt die erfindungsgemäße Zusammensetzung in Form eines Tankmixes vor.

Die verdünnte Zusammensetzung wird üblicherweise durch Versprühen oder Vernebeln angewendet. Zu dem Tankmix können Öle verschiedenen Typs, Netzmittel, Adjuvants, Herbizide, Bakterizide, Fungizide unmittelbar vor der Anwendung (Tankmix) zugesetzt werden. Diese Mittel können zu den erfindungsgemäßen Zusammensetzungen im Gewichtsverhältnis 1:100 bis 100:1, bevorzugt 1:10 bis 10:1 zugemischt werden. Die Pestizidkonzentration im Tankmix kann in größeren Bereichen variiert werden. Im Allgemeinen liegen sie zwischen 0,0001 und 10%, vorzugsweise zwischen 0,01 und 1 %. Die Aufwandmengen liegen bei der Anwendung im Pflanzenschutz je nach Art des gewünschten Effektes zwischen 0,01 und 2,0 kg Wirkstoff pro ha.

Die Verwendung der agrochemischen Formulierungen ist möglich zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

Weiterhin ist die Verwendung der agrochemischen Formulierungen möglich zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man Saatgüter von Nutzpflanzen mit der Zusammensetzung behandelt.

Die vorliegende Erfindung betrifft auch pflanzliches Vermehrungsmaterial enthaltend das Amphiphil. Weiterhin ist die Verwendung der agrochemischen Formulierungen möglich zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man pflanzliche Vermehrungsmaterialien von Nutzpflanzen mit dem erfindungsgemäßen Amphiphil behandelt.

Pflanzliche Vermehrungsmaterialien können vorbeugend zusammen mit oder bereits vor der Aussaat bzw. zusammen mit oder bereits vor dem Umpflanzen mit dem Amphiphil als solche oder mit einer Zusammensetzung enthaltend mindestens eine Amphiphil behandelt werden. Für die Behandlung pflanzlicher Vermehrungsmaterialien, insbesondere Saatgut, werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gele (GF) verwendet. Diese Zusammensetzungen können auf die Vermehrungsmaterialien, insbesondere Saatgut, unverdünnt oder, bevorzugt, verdünnt angewendet werden. Hierbei kann die entsprechende Zusammensetzung 2 bis 10fach verdünnt werden, so dass in den für die Beize zu verwendeten Zusammensetzungen 0,01 to 60% Gew.-%, vorzugsweise 0,1 to 40% Gew.-% Wirkstoff vorhanden sind. Die Anwendung kann vor oder während der Aussaat erfolgen.

Die Behandlung von pflanzlichem Vermehrungsmaterial, insbesondere die Behandlung von Saatgut, sind dem Fachmann bekannt, und erfolgen durch Bestäuben, Beschichten, Pelletieren, Eintauchen oder Tränken des pflanzlichen Vermehrungsmaterial, wobei die Behandlung bevorzugt durch Pelletieren, Beschichten und Bestäuben oder durch Furchenbehandlung erfolgt, so dass z. B. eine vorzeitige Keimung des Saatguts verhindert wird. Die Behandlung mit dem Amphiphil kann optional eine Behandlung mit einem Pestizid umfassen, wobei die Behandlung mit dem Pestizid gleichzeitig, vor oder nach der Behandlung mit dem Amphiphil stattfinden kann.

Für die Saatgutbehandlung werden bevorzugt Suspensionen verwendet. Üblicherweise enthalten solche Zusammensetzungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser. Bei der Behandlung von pflanzlichen Vermehrungsmaterialien, z. B. Staatgut, werden im allgemeinen Wirkstoffmengen von 0,1 bis 1000 g/100 kg Vermehrungsmaterial bzw. Saatgut, bevorzugt 1 bis 1000 g/100 kg, besonders bevorzugt 1 bis 100 g/100 kg, insbesondere 5 bis 100 g/100 kg verwendet.

Eine gleichzeitige gemeinsame oder getrennte Anwendung vom Amphiphil mit mindestens einem Pestizid ist möglich. Gemeinsame Anwendung im Sinne dieser Anmeldung bedeutet, dass der wenigstens ein Amphiphil und der wenigstens Pestizid gleichzeitig am Wirkort (z.B. die zu bekämpfenden planzenschädigenden Pilzen und deren Lebensraum wie befallene Pflanzen, Pflanzenvermehrungsmaterialien, insebesondere Saatgut, Erdböden, Materialien oder Räume sowie die vor Pilzbefall zu schützenden Pflanzen, Pflanzenvermehrungsmaterialien, insbesondere Saatgut, Erdböden, Materialien oder Räume) in eineausreichenden Menge varliegen. Dies kann dadurch erreicht werden, dass man das Amphiphil und mindestens ein Pestizid gemeinsam in einer gemeinsamen Wirkstoffaufbereitung oder in mindestens zwei getrennten Wirkstoffaufbereitungen gleichzeitig ausbringt oder indem man die Stoffe nacheinander am Wirkort appliziert, wobei der zeitliche Abstand der einzelnen Applikationen so gewählt wird, dass der zuerst ausgebrachte Stoff zum Zeitpunkt der Applikation des/der weiteren Wirkstoffs/stoffe in ausreichender Menge am Wirkort vorliegt. Die zeitliche Reihenfolge des Ausbringens der Stoffe ist von untergeordneter Bedeutung.

Die Erfindung betrifft auch ein Amphiphil enthaltend ein hyperverzweigtes Polycarbonat, das verknüpft ist mit mindestens einem linearen oder Kamm-förmigen Polymer, wobei das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis eines polaren, ethylenisch ungesättigten Monomer,
c) ein Polykondensat enthaltend Polyethylenglykol, oder
d) ein Polyethylenglykol
ist, wobei das Polyethylenglykol d) über ein Linker mit dem Polycarbonat vernüpft ist,
wobei der Linker ein Polyisocyanat ist mit einer Funktionalität bezüglish der Isocyanatgrupen von wenigstens 1,5 ist und ausgewählt ist aus aliphatischen, cycloaliphatischen und aromatischen Di- und Polyisocyanaten sowie Isocyanuraten, Allophanaten, Urethdione und Biurete von aliphatischen, cycloaliphatischen und aromatischen Diisocyanaten, und
wobei das Polycarbonat einen Alkohol (B1) enthält, der ein tri- oder höherfunktionelles Polyetherol ist auf Basis von Alkoholen, die mindestens drei OH-Gruppen aufweisen, und C₃-C₂₄ Alkylenoxid.

Geeignete und bevorzugte lineare oder Kamm-förmige Polymere sind wie vorstehend beschrieben. Besonders bevorzugt ist das lineare Polymer eines der vorgenannten Polymere a), b) oder c). In einer weiteren besonders bevorzugten Ausführungsform ist das lineare Polymer eines der vorgenannten Polymere a), c) oder d). Ganz besonders bevorzugt ist das lineare Polymer eines der vorgenannten Polymere a) oder c) Speziell bevorzugt enthält das Kamm-förmige Polymer Polyethylenglykol-mono(meth)acrylat in polymerisierter Form. Bevorzugte Linker ist ein Polyisocyanat.

Das hyperverzweigte Polycarbonat ist üblicherweise erhältlich durch
a) Herstellung eines Kondensationsproduktes (K) durch Umsetzung eines organisches Carbonats (A) oder eines Phosgenderivates mit einem Alkohol (B1), der mindestens drei Hydroxygruppen aufweist, und
b) intermolekularer Umsetzung von K zu dem hyperverzweigten Polycarbonat, wobei das Mengenverhältnis der OH-Gruppen zu den Carbonat- oder Phosgengruppen so gewählt wird, dass K im Mittel entweder i) eine Carbonat- oder Carbamoylchloridgruppe und mehr als eine OH-Gruppe, oder ii) eine OH-Gruppe und mehr als eine Carbonat- oder Carbamoylgruppe aufweisen. Weitere bevorzugte Ausführungsformen des hyperverzweigten Polycarbonats sind vorstehend beschrieben.

Die Erfindung betrifft auch ein Verfahren zur Herstellung des erfindungsgemäßen Amphiphils indem man das Polycarbonat, das lineare oder Kamm-förmigen Polymer und ein Linker umsetzt. Bevorzugte Linker und Umsetzungsbedingungen sind wie vorstehend beschrieben. Bevorzugt ist das Amphiphil erhältlich durch
a) Bereitstellung des hyperverzweigten Polycarbonats und des linearen oder Kamm-förmigen Polymers, und anschließend
b) Verknüpfung der Komponenten mit dem Linker.

Besonders bevorzugt wird das Amphiphil auf diese Weise erhalten. Der Linker ist bevorzugt ein Diisocyanat.

In einer weiteren Ausführungsform betrifft die Erfindung eine Verwendung eines Amphiphils in Zusammensetzungen enthaltend einen Wirkstoff, der in Wasser bei 20 °C zu höchstens 10 g/L löslich ist, wobei das Amphiphil ein hyperverzweigtes Polycarbonat enthält, das verknüpft ist mit mindestens einem linearen oder Kamm-förmigen Polymer, wobei das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis eines polaren, ethylenisch ungesättigten Monomer,
c) ein Polykondensat enthaltend Polyethylenglykol, oder
d) ein Polyethylenglykol
ist, wobei das Polyethylenglykol d) über einen Linker mit dem Polycarbonat verknüpft ist, wobei der Linker ein Polyisocyanat ist mit einer Funktionalität bezüglich der Isocyanatgruppen von wenigstens 1,5 ist und ausgewählt ist aus aliphatischen, cycloaliphatischen und aromatischen Di- und Polyisocyanaten sowie Isocyanuraten, Allophanaten, Urethdione und Biurete von aliphatischen, cycloaliphatischen und aromatischen Diisocyanaten, und
wobei das Polycarbonat einen Alkohol (B1) enthält, der ein tri- oder höherfunkti-onelles Polyetherol ist auf Basis von Alkoholen, die mindestens drei OH-Gruppen aufweisen, und C3-C24 Alkylenoxid.

Bevorzugte Wirkstoffe sind agrochemische Wirkstoffe, kosmetische Wirkstoffe, pharmazeutische Wirkstoffe oder Nahrungsergänzungsmittel (wie Vitamine und Carotinoide), insbesondere agrochemische Wirkstoffe.

Vorteile der vorliegenden Erfindung sind, dass eine hohe Konzentration von Wirkstoff in Lösung gebracht werden kann, dass die Herstellung des Amphiphils aus vorgefertigten Polymeren sehr einfach und großtechnisch mittels einem Linker möglich ist, dass die linearen oder Kamm-förmigen Polymere (insbesondere die statistischen Copolymere und die polaren Homopolymere sowie Polyethylenglykol) sehr einfach zugänglich sind und sogar auf bestimmte Amphiphile optimiert werden können, dass das Amphiphil selbst wasserlöslich oder wasserdispergierbar ist, oder dass es weniger hydrolyseempfindlich ist als viele Polyester. Weiterer Vorteil ist, dass durch den bevorzugten Einsatz eines Polyisocyanats als Linker aus der Reaktion von lsocyanatgruppen zusätzliche Urethan- oder Harnstoff-Bindungen entstehen, wodurch der hydrophobe Anteil des Amphiphils erhöht wird. Dadurch zeigen die erfindungsgemäßen Amphiphile im Vergleich zu anderen modifizierten, hyperverzweigten Polycarbonaten nochmals deutlich verbesserte Solubilisierungseigenschaften.

Nachfolgende Beispiele erläutern die Erfindung ohne sie einzuschränken.

### Beispiele

TMP x 5,2 PO: Umsetzungsprodukt von Trimethylolpropan mit 5,2 molarem Überschuß Propylenoxid (PO).
TMP x 15,7 PO: Umsetzungsprodukt von Trimethylolpropan mit 15,7 molarem Überschuß Propylenoxid.
Gly x 5,7 PO: Umsetzungsprodukt von Glycerin mit 5,7 molarem Überschuß PO.
PE x 5,0 EO: Umsetzungsprodukt von Pentaerythrit mit 5,0 molarem Überschuß Ethylenoxid
DBTL: Dibutylzinndilaurat
IPDI: lsophorondiisocyanat
AIBN: Azo-bis-(isobutyronitril)
PEGMEMA 475: Polyethylenglykolmonomethylethermethacrylat (M = 475 g/mol)

Die hyperverzweigten Polymere wurden per Gelpermeationschromatographie mit einem Refraktometer als Detektor analysiert. Als mobile Phase wurde Dimethylacetamid verwendet, als Standard zur Bestimmung des Molekulargewichts wurde Polymethylmetacrylat (PMMA) eingesetzt. Die Bestimmung der OH-Zahl erfolgte nach DIN 53240, Teil 2. Die Molmassen der linear-dendritischen Copolymere wurden rechnerisch aus dem zahlenmittleren Molekulargewicht des zugrundeliegenden hyperverzweigten Kerns, dessen OH-Zahl und dem gewählten Funktionalisierungsgrad (stöchiometrisches Verhältnis NCO-Gruppen der funktionellen linearen Polymere / verfügbare OH-Gruppen des Kernmoleküls) bestimmt (Annahme einer quantitativen Additionsreaktion der Linker-reaktiven Gruppen an den Linker).

### Synthesebeispiel 1: Hyperverzweigtes Polycarbonat mit terminalen Carbonsäuregruppen (A.1)

3010 g des trifunktionellen Alkohols TMP x 5,2 PO, 1075 g Diethylcarbonat sowie 0,5 g Katalysator KOH wurden vorgelegt. Die Reaktionsmischung wurde zum Sieden erhitzt und so lange gerührt, bis die Siedetemperatur des Reaktionsgemisches durch die Siedekühlung des freiwerdenden Ethanols auf eine konstante Temperatur (ca. 126 °C) gefallen war. Nun wurde Ethanol abdestilliert und die Temperatur des Reaktionsgemisches langsam bis auf 190 °C erhöht. Ethanol wurde in einem gekühlten Rundkolben gesammelt, ausgewogen und der Umsatz so gegenüber dem theoretisch möglichen Vollumsatz prozentual ermittelt. Nach Erreichen eines Umsatzes von 85% wurde die Reaktion durch Zugabe von 85%iger Phoshorsäure auf einen pH-Wert von 7 gebracht. Anschließend wurden bei 130 °C und einem Druck von 100 mbar über 30 min verbleibende flüchtige Bestanteile entfernt und der Ansatz nachfolgend auf Raumtemperatur abgekühlt.

Von dem so erhaltenen Produkt wurden 258 g mit 78 g Bernsteinsäureanhydrid versetzt und auf 130 °C erhitzt. Nach 140 min wurde abgekühlt und das im Rückflusskühler sublimierte Bernsteinsäureanhydrid durch Zugabe von 46 g Aceton wieder der Reaktion zurückgeführt. Unter Abdestillieren des Acetons wurde die Reaktionsmischung während 1 h erneut auf 130 °C erhitzt und weitere 30 min bei dieser Temperatur gehalten. Dann wurde auf Raumtemperatur abgekühlt.

Das Polymer A.1 (Mn = 3100 g/mol; Mw = 61700 g/mol; OH-Zahl: 37 mg KOH/g Polymer; Säurezahl: 135 mg KOH/g Polymer) wurde in Form einer gelb gefärbten, hoch viskosen Flüssigkeit erhalten, welche nach Neutralisierung von 75 % der terminalen Carbonsäuregruppen mit Triethanolamin sehr gut wasserlöslich war. Das Polymer A.1 wurde in dieser teilneutralisierter Form für die weiteren Versuche eingesetzt.

### Synthesebeispiel 2: Hyperverzweigter Polycarbonat-Kern mit terminalen Hydroxylgruppen (A.2)

2298 g TMP x 15,7 PO, 284 g Diethylcarbonat sowie 2 g Katalysator DBTL wurden vorgelegt und zum Sieden erhitzt. Die siedende Reaktionsmischung wurde so lange gerührt (ca 14 h), bis die Siedetemperatur des Reaktionsgemisches durch die Siedekühlung des freiwerdenden Ethanols auf eine konstante Temperatur von ca 143 °C gefallen war. Nun wurde der Rückflusskühler durch eine Destillationsbrücke ersetzt und das bei der Reaktion entstandene Ethanol abdestilliert, wobei die Temperatur des Reaktionsgemisches bis auf 230 °C erhöht wurde. Das Ethanol wurde in einem gekühlten Rundkolben gesammelt, ausgewogen und der Umsatz so gegenüber dem theoretisch möglichen Vollumsatz prozentual ermittelt. Nach dem Erreichen eines Umsatzes von 89% wurde bei einer Temperatur von 160 °C für 1 h Stunde trockener Stickstoff durch die Reaktionsmischung geleitet, um noch vorhandene Restmengen an Monomeren zu entfernen. Danach wurde der Ansatz auf Raumtemperatur abgekühlt.

Das Polymer A.2 (Mn = 2400 g/mol; Mw = 4600 g/mol; OH-Zahl: 87 mg KOH/g Polymer) wurde in Form einer gelb gefärbten, hoch viskosen Flüssigkeit erhalten, welche nicht wasserlöslich war.

### Synthesebeispiel 3: Hyperverzweigter Polycarbonat-Kern mit terminalen Hydroxylgrupen A.3

1149 g des trifunktionellen Alkohols TMP x 15,7 PO, 144 g Diethylcarbonat sowie 1 g Katalysator DBTL wurden wie in Synthesebeispiel 2 umgesetzt. Das Polymer A.3 (Mn = 4200 g/mol; Mw = 14500 g/mol; OH-Zahl: 85 mg KOH/g Polymer) wurde in Form einer gelb gefärbten, hoch viskosen Flüssigkeit erhalten, welche nicht wasserlöslich war.

### Synthesebeispiel 4: Hyperverzweigter Polycarbonat-Kern mit terminalen Hydroxylgruppen (A.4)

2000 g des trifunktionellen Alkohols Gly x 5,7 PO, 562 g Diethylcarbonat sowie 0,4 g Katalysator KOH wurden vorgelegt und solange zum Sieden erhitzt, bis die Siedetemperatur des Reaktionsgemisches durch die Siedekühlung des freiwerdenden Ethanols auf eine konstante Temperatur von ca 113 °C gefallen war. Nun wurde der Rückflusskühler durch eine Destillationsbrücke ersetzt und das bei der Reaktion entstandene Ethanol abdestilliert, wobei die Temperatur des Reaktionsgemisches bis 200 °C erhöht wurde. Ethanol wurde in einem gekühlten Rundkolben gesammelt, ausgewogen und der Umsatz so gegenüber dem theoretisch möglichen Vollumsatz prozentual ermittelt. Nach dem Erreichen eines Umsatzes von 80% wurde das Reaktionsgemisch auf 100 °C abgekühlt und durch Zugabe von 0,45 g 85%iger Phosphorsäure auf einen pH-Wert von 7 eingestellt. Anschließend wurde die Reaktionsmischung wieder auf 200 °C erhitzt und bei dieser Temperatur über einen Zeitraum von 3,5 h trockener Stickstoff durchgeleitet, um noch vorhandene Restmengen an Monomeren zu entfernen. Danach wurde der Ansatz auf Raumtemperatur abgekühlt.

Das Polymer A.4 (Mn = 1700 g/mol; Mw = 3300 g/mol; OH-Zahl: 206 mg KOH/g Polymer) wurde in Form einer gelb gefärbten, hoch viskosen Flüssigkeit erhalten, welche nicht wasserlöslich war.

### Synthesebeispiel 5: Hyperverzweigter Polycarbonat-Kern A.2 funktionalisiert mit PEG-Ketten (Funktionalisierungsgrad 100%, A.5)

Stufe 1 (A.5a): 123,5 g Polyethylenglykolmonomethylether (Mn = 500 g/mol) wurden vorgelegt und im Vakuum bei 80 °C von Wasserresten befreit. Nach dem Abkühlen auf Raumtemperatur wurde der Ansatz unter Stickstoff gesetzt und das Polymer in 123,5 g Butylacetat gelöst. Nun wurden 50,0 g Isophorondiisocyanat zugegeben und das Gemisch auf 50 °C erhitzt. Durch Zugabe von 19 mg Zink-Neodecanoat, gelöst in 1 mL Butylacetat, wurde die Reaktion gestartet und im Verlauf von 3,5 h bei 50 °C bis auf einen NCO-Gehalt von 2,87 % gefahren. Anschließend wurde die Reaktion durch Abkühlen auf -20 °C beendet. Das Umsetzungsprodukt A.5a wurde ohne weitere Aufarbeitung direkt in Stufe 2 eingesetzt.
Stufe 2 (A.5): 30,1 g des hydrophoben hyperverzeigten Polycarbonat-Kerns A.2 wurden vorgelegt und unter Stickstoff mit 71,0 g des Reaktionsgemisches A.5a versetzt. Nun wurde der Ansatz auf 80 °C erhitzt und die Reaktion durch Zugabe von 7 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.5 (Mn = 5070 g/mol) in Form einer gelb gefärbten, hoch viskosen Flüssigkeit erhalten, welche vollständig wasserlöslich war.

### Synthesebeispiel 6: Hyperverzweigter Polycarbonat-Kern A.3 funktionalisiert mit PEG-Ketten (Funktionalisierungsgrad 100%, A.6)

Stufe 1 (A.6a): 247,5 g Polyethylenglykolmonomethylether (Mn = 500 g/mol) wurden vorgelegt und im Vakuum bei 80 °C von Wasserresten befreit. Nach dem Abkühlen auf Raumtemperatur wurde der Ansatz unter Stickstoff gesetzt und das Polymer in 247,5 g Butylacetat gelöst. Nun wurden 100,0 g IPDI zugegeben und das Gemisch auf 50 °C erhitzt. Durch Zugabe von 37 mg Zink-neodecanoat, gelöst in 1 mL Butylacetat, wurde die Reaktion gestartet und imVerlauf von ca. 3 h bei 50 °C bis auf einen NCO- Gehalt von 2,88 % gefahren. Anschließend wurde die Reaktion durch Abkühlen auf -20 °C beendet. Das Umsetzungsprodukt A.6a wurde ohne weitere Aufarbeitung direkt in Stufe 2 eingesetzt.
Stufe 2 (A.6): 25,0 g des hydrophoben hyperverzeigten Polycarbonat-Kerns A.3 wurden vorgelegt und unter Stickstoff in 25,0 g Butylacetat gelöst. Der Ansatz wurde mit 60,25 g des Reaktionsgemischs A.6a versetzt, auf 80 °C erhitzt und die Reaktion durch Zugabe von 80 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.6 (Mn = 8810 g/mol) in Form einer gelb gefärbten, hoch viskosen Flüssigkeit erhalten, welche vollständig wasserlöslich war.

### Synthesebeispiel 7: Hyperverzweigter Polycarbonat-Kern A.3 funktionalisiert mit einem kammartigen PMMA-co-PS-co-PEGMEMA-Copolymer (Funktionalisierungsgrad 100%, A.7)

Stufe 1 (A.7a): 250,0 g Tetrahydrofuran (THF) wurden unter Stickstoff vorgelegt und anschließend unter Rückfluss erhitzt. Innerhalb von 2 h wurde ein Gemisch 1 aus 180,2 g Methylmethacrylat (MMA), 70,3 g Styrol und 214,0 g PEGMEMA 475 sowie zeitgleich innerhalb von 4 h ein Gemisch 2 aus 8,6 g AIBN und 27,0 g Mercaptoethanol, gelöst in 250,0 g THF, mit Hilfe zweier Dosierpumpen langsam dem Ansatz zugeführt. Nach beendeter Zugabe des Gemischs 2 wurde die Reaktionsmischung weitere 16 h unter Rückfluss erhitzt. Nachfolgende Kontrolle der Restmonomere mittels GC ergab einen Anteil an MMA von <1 %, so dass der Ansatz abgekühlt und das Produkt A.7a (Mn = 1030 g/mol) direkt weiter in Stufe 2 eingesetzt wurde.
Stufe 2 (A.7b): 300,0 g des Reaktionsgemischs A.7a wurden vorgelegt und im Vakuum vom Lösungsmittel THF befreit. Nach dem Abkühlen auf Raumtemperatur wurde der Ansatz unter Stickstoff gesetzt und der Rückstand in 152,0 g Butylacetat gelöst. Nun wurden 20,94 g Isophorondiisocyanat zugegeben und das Gemisch auf 50 °C erhitzt. Durch Zugabe von 30 mg Zink-neodecanoat, gelöst in 1 mL Butylacetat, wurde die Reaktion gestartet und imverlauf von ca. 7 h bei 50 °C bis auf einen NCO-Gehalt von 1,09 % gefahren. Anschließend wurde die Reaktion durch Abkühlen auf -20 °C beendet. Das Umsetzungsprodukt A.7b wurde ohne weitere Aufarbeitung direkt in Stufe 3 eingesetzt.
Stufe 3 (A.7): 20,0 g des hydrophoben hyperverzeigten Polycarbonat-Kerns A.3 wurden vorgelegt und unter Stickstoff in 20,0 g Butylacetat gelöst. Nun wurde der Ansatz mit 127,0 g des Reaktionsgemischs A.7b versetzt, auf 80 °C erhitzt und die Reaktion durch Zugabe von 84 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.7 (Mn = 12200 g/mol) in Form einer gelb gefärbten, hoch viskosen Flüssigkeit erhalten, welche vollständig wasserlöslich war.

### Synthesebeispiel 8: Hyperverzweigter Polycarbonat-Kern funktionalisiert mit einem kammartigen PVP-co-PLaurylacrylat-co-PEGMEMA-Copolymer (Funktionalisierungsgrad 30%, A.8)

Stufe 1 (A.8a): 100,0 g THF wurden unter Stickstoff vorgelegt und anschließend unter Rückfluss erhitzt. Innerhalb von 3 h wurde ein Gemisch 1 aus 155,9 g Laurylacrylat, 144,2 g N-Vinylpyrrolidon und 163,3 g PEGMEMA 475, gelöst in 200,0 g THF, sowie zeitgleich innerhalb von 4 h ein Gemisch 2 aus 8,8 g AIBN und 27,8 g Mercaptoethanol, gelöst in 200,0 g THF, mit Hilfe zweier Dosierpumpen langsam dem Ansatz zugeführt. Nach beendeter Zugabe des Gemischs 2 wurde die Reaktionsmischung weitere 18 h unter Rückfluss erhitzt. Nachfolgende Kontrolle der Restmonomere mittels GC ergab einen Anteil an Laurylacrylat von <1 %, so dass der Ansatz abgekühlt und das Produkt A.8a (Mn = 1000 g/mol) direkt weiter in Stufe 2 eingesetzt wurde.
Stufe 2 (A.8b): 278,4 g des Reaktionsgemischs A.8a wurden vorgelegt und im Vakuum vom Lösungsmittel THF befreit. Nach dem Abkühlen auf Raumtemperatur wurde der Ansatz unter Stickstoff gesetzt und der Rückstand in 140,0 g Butylacetat gelöst. Nun wurden 20,0 g Isophorondiisocyanat zugegeben und das Gemisch auf 50 °C erhitzt. Durch Zugabe von 21 mg Zink-neodecanoat, gelöst in 1 mL Butylacetat, wurde die Reaktion gestartet und im Verlauf von 6 h bei 60 °C sowie insgesamt 16 h bei Raumtemperatur bis auf einen NCO-Gehalt von 1,16 % gefahren. Anschließend wurde die Reaktion durch Abkühlen auf -20 °C beendet. Das Umsetzungsprodukt A.8b wurde ohne weitere Aufarbeitung direkt in Stufe 3 eingesetzt.
Stufe 3 (A.8): 6,0 g des hydrophoben hyperverzeigten Polycarbonat-Kerns A.3 wurden vorgelegt und unter Stickstoff in 6,0 g Butylacetat gelöst. Nun wurde der Ansatz mit 11,4 g des Reaktionsgemischs A.8b versetzt, auf 80 °C erhitzt und die Reaktion durch Zugabe von 12 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.8 (Mn = 6540 g/mol) in Form einer gelb gefärbten, hoch viskosen Flüssigkeit erhalten, welche vollständig wasserlöslich war.

### Synthesebeispiel 9: Hyperverzweigter Polycarbonat-Kern funktionalisiert mit einem kammartigen PVP-co-PLaurylacrylat-co-PEGMEMA-Copolymer (Funktionalisierungsgrad 50%, A.9)

Stufe 1 (A.9a): 100,0 g THF wurden unter Stickstoff vorgelegt und anschließend unter Rückfluss erhitzt. Innerhalb von 3 h wurde ein Gemisch 1 aus 155,9 g Laurylacrylat, 144,2 g N-Vinylpyrrolidon und 163,3 g PEGMEMA 475, gelöst in 200,0 g THF, sowie zeitgleich innerhalb von 4 h ein Gemisch 2 aus 8,8 g AIBN und 27,8 g Mercaptoethanol, gelöst in 200,0 g THF, mit Hilfe zweier Dosierpumpen langsam dem Ansatz zugeführt. Nach beendeter Zugabe des Gemischs 2 wurde die Reaktionsmischung weitere 18 h unter Rückfluss erhitzt. Nachfolgende Kontrolle der Restmonomere mittels GC ergab einen Anteil an Laurylacrylat von <1 %, so dass der Ansatz abgekühlt und das Produkt A.9a (Mn = 1000 g/mol) direkt weiter in Stufe 2 eingesetzt wurde.
Stufe 2 (A.9b): 278,4 g des Reaktionsgemischs A.9a wurden vorgelegt und im Vakuum vom Lösungsmittel THF befreit. Nach dem Abkühlen auf Raumtemperatur wurde der Ansatz unter Stickstoff gesetzt und der Rückstand in 140,0 g Butylacetat gelöst. Nun wurden 20,0 g Isophorondiisocyanat zugegeben und das Gemisch auf 50 °C erhitzt. Durch Zugabe von 21 mg Zink-neodecanoat, gelöst in 1 mL Butylacetat, wurde die Reaktion gestartet und im Verlauf von 6 h bei 60 °C sowie insgesamt 16 h bei Raumtemperatur bis auf einen NCO-Gehalt von 1,16 % gefahren. Anschließend wurde die Reaktion durch Abkühlen auf -20 °C beendet. Das Umsetzungsprodukt A.9b wurde ohne weitere Aufarbeitung direkt in Stufe 3 eingesetzt.
Stufe 3 (A.9): 6,0 g des hydrophoben hyperverzeigten Polycarbonat-Kerns A.3 wurden vorgelegt und unter Stickstoff in 6,0 g Butylacetat gelöst. Nun wurde der Ansatz mit 19,2 g des Reaktionsgemischs A.9b versetzt, auf 80 °C erhitzt und die Reaktion durch Zugabe von 13 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.9 (Mn = 8110 g/mol) in Form einer gelb gefärbten, hoch viskosen Flüssigkeit erhalten, welche vollständig wasserlöslich war.

### Synthesebeispiel 10: Hyperverzweigter Polycarbonat-Kern A.3 funktionalisiert mit PEG-b-Polycaprolacton Blockcopolymer (Funktionalisierungsgrad 100%, A.10)

Stufe 1 (A.10a): 150,0 g Polyethylenglykolmonomethylether (Mn = 500 g/mol) wurden vorgelegt und im Vakuum bei 90 °C von Wasserresten befreit. Nach dem Abkühlen auf Raumtemperatur wurde der Ansatz unter Stickstoff gesetzt und das Polymer mit 205,0 g ε-Caprolacton versetzt. Das Gemisch wurde auf 90 °C erwärmt und die ringöffnende Polymerisation des Caprolactons durch Zugabe von 355 mg Butylzinn-tris(2-ethylhexanoat) gestartet. Der Ansatz wurde weitere 18 h bei 90 °C erhitzt und nach beendeter Reaktion auf Raumtemperatur abgekühlt. Das so erhaltene, OH-terminierte Blockcopolymer A.10a (Mn = 1180 g/mol) wurde ohne weitere Aufreinigung direkt in Stufe 2 eingesetzt.
Stufe 2 (A.10b): 200,0 g des Blockcopolymers A.10a wurden vorgelegt, unter Stickstoff gesetzt und mit 34,1 g Isophorondiisocyanat versetzt. Das Gemisch wurde auf 50 °C erhitzt. Durch Zugabe von 30 mg Zink-neodecanoat, gelöst in 1 mL Butylacetat, wurde die Reaktion gestartet und im Verlauf von 4 h bei 50 °C bis auf einen NCO-Gehalt von 2,23 % gefahren. Anschließend wurde die Reaktion durch Abkühlen auf -20 °C beendet. Das Umsetzungsprodukt A.10b wurde ohne weitere Aufarbeitung direkt in Stufe 3 eingesetzt.
Stufe 3 (A.10): 7,0 g des hydrophoben hyperverzeigten Polycarbonat-Kerns A.3 wurden vorgelegt und unter Stickstoff in 10,0 g Butylacetat gelöst. Nun wurde der Ansatz mit 20,0 g des Reaktionsgemischs A.10b versetzt, auf 80 °C erhitzt und die Reaktion durch Zugabe von 27 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel im Vakuum entfernt. Schließlich wurde das linear-dendritische Copolymer A.10 (Mn = 13190 g/mol) in Form einer gelb gefärbten, hoch viskosen Flüssigkeit erhalten, welche vollständig wasserlöslich war.

### Synthesebeispiel 11: Hyperverzweigter Polycarbonat-Kern A.4 funktionalisiert mit PEG-b-PUR Blockcopolymer (Funktionalisierungsgrad 100%, A.11)

Stufe 1 (A.11a): Es wurden 23,4 g Neopentylglykol und 20,3 g 1,3-Butandiol in 100,0 g THF gelöst. Der Ansatz wurde unter Stickstoff gesetzt und mit 100,8 g Hexamethylendiisocyanat (HDI), gelöst in 44,5 g THF, versetzt. Durch Zugabe von 140 mg Zink-neodecanoat, gelöst in 1 mL THF, wurde die exotherme Reaktion gestartet, was sich an einem Temperaturanstieg auf ca. 50 °C manifestierte. Im Folgenden wurde eine Innentemperatur von 50 °C aufrechterhalten und der Ansatz im Verlauf von 7,5 h bei 50 °C bis auf einen NCO-Gehalt von 4,40 % gefahren. Anschließend wurde eine Lösung von 300,0 g Polyethylenglykolmonomethylether (Mn = 2000 g/mol) in 300,0 g THF dem Reaktionsgemisch hinzugefügt und dieses weitere 4 h auf 50 °C erhitzt. Nach dem Erreichen eines NCO-Gehalts von 0,79 % wurde die Reaktion durch Abkühlen auf -20 °C beendet. Das Umsetzungsprodukt A.11a (Mn = 2960 g/mol) wurde ohne weitere Aufarbeitung direkt in Stufe 2 eingesetzt.
Stufe 2 (A.11): 3,0 g des hydrophoben hyperverzeigten Polycarbonat-Kerns A.4 wurden vorgelegt und unter Stickstoff mit 75,5 g des Umsetzungsprodukts A.11a versetzt, woraufhin eine klare Lösung resultierte. Nun wurde der Ansatz auf 50 °C erhitzt und die Reaktion durch Zugabe von 2 mg DBTL, gelöst in 1 mL Butylacetat, gestartet. Nach dem vollständigen Umsatz aller NCO-Gruppen (NCO-Gehalt 0 %) wurde der Ansatz abgekühlt und das Lösungsmittel THF im Vakuum entfernt. Schließlich wurde das linear-dendritische Copolymer A.11 (Mn = 20350 g/mol) in Form einer gelb gefärbten, hoch viskosen Flüssigkeit erhalten, welche vollständig wasserlöslich war.

### Synthesebeispiel 12: Herstellung eines Polycarbonats mit polarem Kern und terminalen OH-Gruppen, (A.12)

2000 g des tetrafunktionellen Alkohols PE x 5,0 EO, 665 g Diethylcarbonat sowie 0,4 g Katalysator KOH wurden vorgelegt und solange zum Sieden erhitzt (ca. 5 h), bis die Siedetemperatur des Reaktionsgemisches durch die Siedekühlung des freiwerdenden Ethanols auf eine konstante Temperatur von ca 105 °C gefallen war. Nun wurde der Rückflusskühler durch eine Destillationsbrücke ersetzt und das bei der Reaktion entstandene Ethanol abdestilliert, wobei die Temperatur des Reaktionsgemisches bis auf 190 °C erhöht wurde. Ethanol wurde in einem gekühlten Rundkolben gesammelt, ausgewogen und der Umsatz so gegenüber dem theoretisch möglichen Vollumsatz prozentual ermittelt. Nach dem Erreichen eines Umsatzes von 90% wurde das Reaktionsgemisch auf 100 °C abgekühlt und durch Zugabe von 0,4 g 85%iger Phosphorsäure auf einen pH-Wert < 7 eingestellt. Anschließend wurde die Reaktionsmischung wieder auf 200 °C erhitzt und bei dieser Temperatur über einen Zeitraum von 5 h trockener Stickstoff durchgeleitet, um noch vorhandene Restmengen an Monomeren zu entfernen. Danach wurde der Ansatz auf Raumtemperatur abgekühlt. Das Polymer A.12 (Mn = 3200 g/mol; Mw = 22100 g/mol; OH-Zahl: 335 mg KOH/g Polymer) wurde in Form einer gelb gefärbten, hoch viskosen Flüssigkeit erhalten, welche wasserlöslich war.

### Synthesebeispiel 13: Hyperverzweigter Polycarbonat-Kern funktionalisiert mit PEG-Ketten (A.13)

2000 g TMP x 15,7 PO, 247 g Diethylcarbonat sowie 1,7 g Katalysator DBTL wurden wie in Synthesebeispiel 2 umgesetzt zu einem hyperverzweigten Polycarbonat-Kern (Mn = 3000 g/mol, Mw = 6200 g/mol, OH-Zahl = 87 mg KOH/g). Der so erhaltenen Polycarbonat-Kern wurde mit Ethylenoxid ethoxyliert durch Umsetzung im Gewichtsverhältnis Kern zu Ethylenoxid von 1 zu 1. Dazu wurde der Kern in einem Druckautoklaven vorgelegt und mit einer 50 %igen wässrigen KOH-Lösung versetzt. Nachdem die Reaktionsmischung mit Stickstoff inertisiert worden war, wurde der Ansatz unter Vakuum bei 120 °C von Wasserresten befreit. Anschließend wurden der Reaktionsmischung Ethylenoxid zugefügt. Nach beendeter Ethylenoxid-Dosierung und Erreichen eines konstanten Reaktordrucks wurden nicht umgesetztes Ethylenoxid und andere flüchtige Bestandteile im Vakuum abdestilliert und das ethoxylierte Polymer aus dem Polymerisationsreaktor entnommen und wie üblich aufgearbeitet. Man erhielt das linear-dendritische Copolymer A.13 (OH-Zahl 47 mgKOH/g Polymer) in Form einer gelb gefärnten, hoch viskosen Flüssigkeit, welche vollständig wasserlöslich war.

### Solubilisierungsexperimente:

Die Wellenlängen der UV-spektroskopischen Messungen (falls anwendbar) sind in Tabelle 1 zusammengestellt.

**Tabelle 1:**

| Zu lösende Verbindung | Wellenlänge der UV-Messung [nm] |
|---|---|
| Piroxicam | 356 |
| Carbamazepin | 286 |
| Estradiol | 282 |
| Clotrimazol | HPLC-Analytik |
| Pyren | 334 |
| Pyraclostrobin | 277 |
| Fipronil | 280 |

### Allgemeine Arbeitsvorschrift 1 für Solubilisierungsexperimente mit Piroxicam, Carbamazepin, Estradiol und Clotrimazol

In einem 50 mL Becherglas wurden ca. 2 g Polymer eingewogen. Anschließend wurde dem Ansatz jeweils 0,2 g Wirkstoff zugewogen, um eine übersättigte Lösung zu erhalten. Nun wurde soviel Phosphatpuffer pH 7,0 zugegeben, dass ein Masseverhältnis Polymer : Phosphatpuffer von 1 : 9 vorlag. Das Gemisch wurde nun 72 h bei Raumtemperatur mit Hilfe eines Magnetrührers gerührt. Nach einstündiger Ruhezeit wurde nicht solubilisierter Wirkstoff durch Filtration abgetrennt. Die so erhaltene, klare oder opake Lösung wurde anschließend auf ihren Wirkstoffgehalt mittels UV-Spektroskopie oder HPLC untersucht.

**Tabelle 2:**

| Löslichkeit [mg/l] in Gegenwart von | Piroxicam | Carbamazepin | Estradiol | Clotrimazol |
|---|---|---|---|---|
| Ohne Polymer^{a)} | 420 | 140 | <100 | <100 |
| Polymer A.1 | 1600 | 1500 | 400 | 3600 |
| Polymer A.5 | 4400 | 1600 | 700 | 700 |
| Polymer A.6 | 3100 | 1250 | 1080 | 2430 |
| Polymer A.7 | 2410 | 1390 | 1190 | 1830 |
| Polymer A.8 | 2430 | 1440 | 1950 | 2160 |
| Polymer A.9 | 2670 | 1230 | 2360 | 1620 |

| | | | | |
|---|---|---|---|---|
| a) nicht erfindungsgemäß | | | | |

Allgemeine Arbeitsvorschrift 2 für Solubilisierungsexperimente mit Pyren, Pyraclostrobin und Fipronil

In einem 50 mL Becherglas wurden ca. 100 mg Polymer eingewogen und in 9,900 g dest. Wasser gelöst. Anschließend wurde dem Ansatz jeweils 100 mg Wirkstoff zugewogen, um eine übersättigte Lösung zu erhalten. Das Gemisch wurde nun 24 h bei Raumtemperatur mit Hilfe eines Magnetrührers gerührt. Nach einstündiger Ruhezeit wurde nicht solubilisierter Wirkstoff durch Zentrifugieren abgetrennt. Die so erhaltene, klare oder opake Lösung wurde anschließend auf ihren Wirkstoffgehalt mittels UV-Spektroskopie untersucht

**Tabelle 3:**

| Löslichkeit [mg/l] in Gegenwart von | Pyren | Pyraclostrobin | Fipronil |
|---|---|---|---|
| Ohne Polymer^{a)} | 0,1 | 22,5 | 3 |
| Polymer A.1 | 132 | 626 | 79 |
| Polymer A.5 | 143 | 765 | 312 |
| Polymer A.6 | 172 | n.b. | 240 |
| Polymer A.7 | 263 | 1148 | 462 |
| Polymer A.8 | 317 | n.b. | 828 |
| Polymer A.9 | 283 | n.b. | 667 |
| Polymer A.10 | 280 | n.b. | 654 |
| Polymer A.11 | 25 | 225 | n.b. |
| Polymer A.12^{a)} | <1 | <30 | n.b. |

| | | | |
|---|---|---|---|
| n.b.: nicht bestimmt; a) nicht erfindungsgemäß | | | |

Vergleich der Solubilisierungseigenschaften von Kern, Schale, Kern/Schale-Blend und erfindungsgemäßem linear-dendritischem Blockcopolymer Die Löslichkeit wurde wie in der Arbeitsvorschrift 2 beschrieben bestimmt.

**Tabelle 4a:**

| Löslichkeit [mg/l] in Gegenwart von | Pyren | Fipronil | Wasserlöslichkeit des Polymers |
|---|---|---|---|
| Ohne Polymer a) | 0,1 | 3 | - |
| Polymer A.5 | 143 | 312 | Ja |
| Polymer A.2 (nur Kern) ^{a)} | - | - | Nein |
| PEG-monomethylether (nur Schale) ^{a)} | 3 | 6 | Ja |
| Polymer A.2 + PEG-monomethylether (Mischung aus Kern + Schale) ^{a)} | - | - | Nein (ungelöste Anteile) |

| | | | |
|---|---|---|---|
| a) nicht erfindungsgemäß | | | |

Tabelle 4a zeigt, dass das erfindungsgemäße, funktionalisierte Polycarbonat A.5 (A.2 funktionalisiert mit PEG-monomethylether) höhrere Solubilisierungskapazitäten aufweist als die einzelnen Bestandteile, d.h. das Kernpolymer (A.2), das Schalenpolymer (PEG-monomethylether) oder die Mischung (d.h. keine kovalente Verknüpfung) von Kernpolymer und Schalenpolymer.

**Tabelle 4b:**

| Löslichkeit [mg/l] in Gegenwart von | Pyren | Fipronil | Wasserlöslichkeit des Polymers |
|---|---|---|---|
| Ohne Polymer ^{a)} | 0,1 | 3 | - |
| Polymer A.8 | 317 | 828 | Ja |
| Polymer A.3 (nur Kern) ^{a)} | - | - | Nein |
| Polymer A.8a (nur Schale) ^{a)} | 171 | 366 | Ja |
| Polymer A.3 + Polymer A.8a (Mischung aus Kern + Schale) ^{a)} | - | - | Nein (ungelöste Anteile) |

| | | | |
|---|---|---|---|
| a) nicht erfindungsgemäß | | | |

Tabelle 4b zeigt, dass das erfindungsgemäße, funktionalisierte Polycarbonat A.8 (A.3 funktionalisiert mit A.8a) höhere Solubilisierungskapazitäten aufweist als die einzelnen Bestandteile, d.h. das Kernpolymer (A.3), das Schalenpolymer (Polymer A.8a) oder die Mischung (d.h. keine kovalente Verknüpfung) von Kernpolymer und Schalenpolymer.

## Patentansprüche

1. Zusammensetzung enthaltend ein Amphiphil und einen Wirkstoff, der in Wasser bei 20 °C zu höchstens 10 g/L löslich ist, **dadurch gekennzeichnet, dass** das Amphiphil ein hyperverzweigtes Polycarbonat enthält, das verknüpft ist mit mindestens einem linearen oder Kamm-förmigen Polymer und/oder mindestens einer funktionalen C₁-C₂₄-Einheit enthaltend eine Säuregruppe, eine Aminogruppe oder mindestens zwei Hydroxygruppen,
wobei das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis eines polaren, ethylenisch ungesättigten Monomers, oder
c) ein Polykondensat oder Polyadditionsprodukt enthaltend Polyethylenglykol, oder
d) ein Polyethylenglykol
ist, wobei das Polyethylenglykol d) über einen Linker mit dem Polycarbonat vernüpft ist, und
wobei der Linker ein Polyisocyanat mit einer Funktionalität bezüglich der Isocyanatgruppen von wenigstens 1,5 ist und ausgewählt aus aliphatischen, cycloaliphatischen und aromatischen Di- und Polyisocyanaten sowie lsocyanuraten, Allophanaten, Urethdione und Biurete von aliphatischen, cycloaliphatischen und aromatischen Diisocyanaten,
wobei die funktionalen C₁-C₂₄-Einheit erhältlich ist durch Umsetzung des hyperverzweigten Polycarbonats mit einem zyklischen Carbonsäureanhydrid; oder die funktionalen C₁-C₂₄-Einheit Hydroxycarbonsäuren, Aminocarbonsäuren, Hydroxysulfonsäuren, Hydroxysulfate, Aminosulfonsäuren, Aminosulfate, Hydroxyamine, Polyamine oder Polyole sind, die kovalent mittels dem Linker mit dem Polycarbonat verknüpft sind, und
wobei das Polycarbonat einen Alkohol (B1) enthält, der ein tri- oder höherfunktionelles Polyetherol ist auf Basis von Alkoholen, die mindestens drei OH-Gruppen aufweisen, und C₃-C₂₄ Alkylenoxid.

2. Zusammensetzung nach Anspruch 1, **dadurch gekennzeichnet, dass** das Polyethylenglykol ein Polyethylenglykol oder Polyethylenglykolmonoalkylether ist mit einer Molmasse Mn von mindestens 200 g/mol.

3. Zusammensetzung nach Anspruch 1 oder 2, **dadurch gekennzeichnet, dass** das polare, ethylenisch ungesättigte Monomer Vinylpyrrolidon, (Meth)Acrylsäure, ein Sulfonsäure-haltiges Monomer, ein Amino-funktionelles Monomer, oder ein (Meth)Acrylsäureester von einem Polyethylenglykolderivat ist.

4. Zusammensetzung nach einem der Ansprüche 1 bis 3, **dadurch gekennzeichnet, dass** das Kamm-förmige Polymer Polyethylenglykol-mono(meth)acrylat oder Allylalkoholalkoxylat in polymerisierter Form enthält.

5. Zusammensetzung nach einem der Ansprüche 1 bis 4, **dadurch gekennzeichnet, dass** die funktionale C₁-C₂₄-Einheit mittels des Linkers kovalent verknüpft wird und ausgewählt ist aus Hydroxycarbonsäuren, Aminocarbonsäuren, Hydroxysulfonsäuren, Hydroxysulfate, Aminosulfonsäuren oder Aminosulfate, Hydroxyamine (wie Diethanolamin), Polyamine (z.B. Diethylentetramin), oder Polyole (z.B. Glycerin, Trimethylolpropan, Pentaerythrit).

6. Zusammensetzung nach einem der Ansprüche 1 bis 5, **dadurch gekennzeichnet, dass** das Amphiphil ein hyperverzweigtes Polycarbonat enthält, das verknüpft ist mit mindestens einem linearen oder Kamm-förmigen Polymer.

7. Zusammensetzung nach einem der Ansprüche 1 bis 6, **dadurch gekennzeichnet, dass** der Wirkstoff einen agrochemischen oder pharmazeutischen Wirkstoff enthält.

8. Amphiphil enthaltend ein hyperverzweigtes Polycarbonat, das verknüpft ist mit mindestens einem linearen oder Kamm-förmigen Polymer, **dadurch gekennzeichnet, dass** das lineare Polymer
a) ein Homopolymer oder statistisches Copolymer enthaltend ein polares, ethylenisch ungesättigtes Monomer,
b) ein Blockpolymer enthaltend einen Block aus Polyethylenglykol oder auf Basis eines polaren, ethylenisch ungesättigten Monomers,
c) ein Polykondensat oder Polyadditionsprodukt enthaltend Polyethylenglykol, oder
d) ein Polyethylenglykol
ist, wobei das Polyethylenglykol d) über einen Linker mit dem Polycarbonat verknüpft ist,
wobei der Linker ein Polyisocyanat ist mit einer Funktionalität bezüglich der Isocyanatgruppen von wenigstens 1,5 ist und ausgewählt ist aus aliphatischen, cycloaliphatischen und aromatischen Di- und Polyisocyanaten sowie lsocyanuraten, Allophanaten, Urethdione und Biurete von aliphatischen, cycloaliphatischen und aromatischen Diisocyanaten, und
wobei das Polycarbonat einen Alkohol (B1) enthält, der ein tri- oder höherfunktionelles Polyetherol ist auf Basis von Alkoholen, die mindestens drei OH-Gruppen aufweisen, und C₃-C₂₄ Alkylenoxid.

9. Amphiphile nach Anspruch 8, **dadurch gekennzeichnet, dass** das Polycarbonat erhältlich ist durch
a) Herstellung eines Kondensationsproduktes (K) durch Umsetzung eines organisches Carbonats (A) oder eines Phosgenderivates mit dem Alkohol (B1), der mindestens drei Hydroxygruppen aufweist, und
b) intermolekularer Umsetzung von K zu dem hyperverzweigten Polycarbonat,
wobei das Mengenverhältnis der OH-Gruppen zu den Carbonat- oder Phosgengruppen so gewählt wird, dass K im Mittel entweder i) eine Carbonat- oder Carbamoylchloridgruppe und mehr als eine OH-Gruppe, oder ii) eine OH-Gruppe und mehr als eine Carbonat- oder Carbamoylgruppe aufweist.

10. Amphiphil nach Anspruch 8 oder 9, **dadurch gekennzeichnet, dass** das Kamm-förmige Polymer Polyethylenglykol-mono(meth)acrylat in polymerisierter Form enthält.

11. Amphiphil nach einem der Ansprüche 8 bis 10, **dadurch gekennzeichnet, dass** das Polyethylenglykol ein Polyethylenglykol oder Polyethylenglykolmonoalkylether ist mit einer Molmasse Mn von mindestens 200 g/mol.

12. Verfahren zur Herstellung des Amphiphils gemäß einem der Ansprüche 8 bis 11, indem man das Polycarbonat, das lineare oder Kamm-förmige Polymer und einen Linker umsetzt.

13. Verwendung des Amphiphils gemäß einem der Ansprüche 8 bis 11 in Zusammensetzungen enthaltend einen Wirkstoff, der in Wasser bei 20 °C zu höchstens 10 g/L löslich ist, zur Solubilisierung des Wirkstoffs in wässrigem Medium.

14. Verwendung der Amphiphils gemäß einem der Ansprüche 8 bis 11 zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man das Amphiphil auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

15. Pflanzliches Vermehrungsmaterial enthaltend das Amphiphil gemäß einem der Ansprüche 8 bis 11.

## Claims

1. A composition comprising an amphiphile and an active ingredient whose solubility in water at 20°C is not more than 10 g/L, wherein the amphiphile comprises a hyperbranched polycarbonate which is joined to at least one linear or comb-type polymer and/or to at least one functional C₁-C₂₄ unit comprising an acid group, an amino group or at least two hydroxyl groups,
the linear polymer being
a) a homopolymer or random copolymer comprising a polar ethylenically unsaturated monomer,
b) a block polymer comprising a block of polyethylene glycol or based on a polar ethylenically unsaturated monomer, or
c) a polycondensate or polyaddition product comprising polyethylene glycol, or
d) a polyethylene glycol,
the polyethylene glycol d) being joined to the polycarbonate via a linker, and
the linker being a polyisocyanate having a functionality in respect of the isocyanate groups of at least 1.5 and being selected from aliphatic, cycloaliphatic, and aromatic di- and polyisocyanates and also isocyanurates, allophanates, uretdiones, and biurets of aliphatic, cycloaliphatic, and aromatic diisocyanates,
the functional C₁-C₂₄ unit being obtainable by reacting the hyperbranched polycarbonate with a cyclic carboxylic anhydride; or the functional C₁-C₂₄ unit being hydroxycarboxylic acids, aminocarboxylic acids, hydroxysulfonic acids, hydroxysulfates, aminosulfonic acids, aminosulfates, hydroxyamines, polyamines, or polyols, which are linked to the polycarbonate covalently by means of the linker, and
the polycarbonate comprising an alcohol (B1) which is a trifunctional or higher polyfunctional polyetherol based on alcohols which have at least three OH groups, and C₃-C₂₄ alkylene oxide.

2. The composition according to claim 1, wherein the polyethylene glycol is a polyethylene glycol or polyethylene glycol monoalkyl ether having a molar mass Mn of at least 200 g/mol.

3. The composition according to claim 1 or 2, wherein the polar ethylenically unsaturated monomer is vinylpyrrolidone, (meth)acrylic acid, a sulfo-containing monomer, an amino-functional monomer or a (meth)acrylic ester of a polyethylene glycol derivative.

4. The composition according to any of claims 1 to 3, wherein the comb-type polymer comprises polyethylene glycol mono(meth)acrylate or allyl alcohol alkoxylate in polymerized form.

5. The composition according to any of claims 1 to 4, wherein the functional C₁-C₂₄ unit is linked covalently by means of the linker and is selected from hydroxycarboxylic acids, aminocarboxylic acids, hydroxysulfonic acids, hydroxysulfates, aminosulfonic acids or aminosulfates, hydroxyamines (such as diethanolamine), polyamines (e.g., diethylenetetramine), or polyols (e.g., glycerol, trimethylolpropane, pentaerythritol).

6. The composition according to any of claims 1 to 5, wherein the amphiphile comprises a hyperbranched polycarbonate which is joined to at least one linear or comb-type polymer.

7. The composition according to any of claims 1 to 6, wherein the active ingredient comprises an active agrochemical or pharmaceutical ingredient.

8. An amphiphile comprising a hyperbranched polycarbonate which is joined to at least one linear or comb-type polymer, wherein the linear polymer is
a) a homopolymer or random copolymer comprising a polar ethylenically unsaturated monomer,
b) a block polymer comprising a block of polyethylene glycol or based on a polar ethylenically unsaturated monomer,
c) a polycondensate or polyaddition product comprising polyethylene glycol, or
d) a polyethylene glycol,
the polyethylene glycol d) being joined to the polycarbonate via a linker,
the linker being a polyisocyanate having a functionality in respect of the isocyanate groups of at least 1.5 and being selected from aliphatic, cycloaliphatic, and aromatic di- and polyisocyanates and also isocyanurates, allophanates, uretdiones, and biurets of aliphatic, cycloaliphatic, and aromatic diisocyanates, and
the polycarbonate comprising an alcohol (B1) which is a trifunctional or higher polyfunctional polyetherol based on alcohols which have at least three OH groups, and C₃-C₂₄ alkylene oxide.

9. The amphiphile according to claim 8, wherein the polycarbonate is obtainable by
a) preparing a condensation product (K) by reacting an organic carbonate (A) or a phosgene derivative with the alcohol (B1) which has at least three hydroxyl groups, and
b) intermolecularly converting K to the hyperbranched polycarbonate,
the quantitative ratio of the OH groups to the carbonate or phosgene groups being selected such that K has an average of either i) one carbonate or carbamoyl chloride group and more than one OH group, or ii) one OH group and more than one carbonate or carbamoyl group.

10. The amphiphile according to claim 8 or 9, wherein the comb-type polymer comprises polyethylene glycol mono(meth)acrylate in polymerized form.

11. The amphiphile according to any of claims 8 to 10, wherein the polyethylene glycol is a polyethylene glycol or polyethylene glycol monoalkyl ether having a molar mass Mn of at least 200 g/mol.

12. A process for preparing the amphiphile according to any of claims 8 to 11, by reacting the polycarbonate, the linear or comb-type polymer, and a linker.

13. The use of the amphiphile according to any of claims 8 to 11 in a composition comprising an active ingredient whose solubility in water at 20°C is not more than 10 g/L, for solubilizing the active ingredient in aqueous medium.

14. The use of the amphiphile according to any of claims 8 to 11, for controlling phytopathogenic fungi and/or unwanted plant growth and/or unwanted insect or mite infestation and/or for regulating the growth of plants, the amphiphile being caused to act on the respective pests, their habitat or the plants to be protected from the respective pest, or on the soil and/or on unwanted plants and/or the crop plants and/or their habitat.

15. A plant propagation material comprising the amphiphile according to any of claims 8 to 11.

## Revendications

1. Composition contenant un amphiphile et une substance active, qui est soluble dans l'eau à 20°C à raison d'au plus 10 g/1, **caractérisée en ce que** l'amphiphile contient un polycarbonate hyperramifié, qui est lié à au moins un polymère linéaire ou en forme de peigne et/ou à au moins une unité fonctionnelle en C₁-C₂₄ contenant un groupe acide, un groupe amino ou au moins deux groupes hydroxy,
le polymère linéaire étant
a) un homopolymère ou un copolymère statistique contenant un monomère polaire, éthyléniquement insaturé,
b) un polymère à blocs contenant un bloc de polyéthylèneglycol ou à base d'un monomère polaire, éthyléniquement insaturé ou
c) un polycondensat ou un produit de polyaddition contenant du polyéthylèneglycol ou
d) un polyéthylèneglycol,
le polyéthylèneglycol d) étant lié via un lieur au polycarbonate et
le lieur étant un polyisocyanate présentant une fonctionnalité, en ce qui concerne les groupes isocyanate, d'au moins 1,5 et choisi parmi les diisocyanates et les polyisocyanates aliphatiques, cycloaliphatiques et aromatiques ainsi que les isocyanurates, les allophanates, les uretdiones et les biurets de diisocyanates aliphatiques, cycloaliphatiques et aromatiques,
l'unité fonctionnelle en C₁-C₂₄ étant obtenue par transformation du polycarbonate hyperramifié avec un anhydride d'acide carboxylique ; ou l'unité fonctionnelle en C₁-C₂₄ étant des acides hydroxycarboxyliques, des acides aminocarboxyliques, des acides hydroxysulfoniques, des hydroxysulfates, des acides aminosulfoniques, des aminosulfates, des hydroxyamines, des polyamines ou des polyols, qui sont liés par covalence au moyen du lieur au polycarbonate et
le polycarbonate contenant un alcool (B1), qui est un polyétherol trifonctionnel ou de fonctionnalité supérieure à base d'alcools, qui présentent au moins trois groupes OH, et d'oxyde d'alkylène en C₃-C₂₄.

2. Composition selon la revendication 1, **caractérisée en ce que** le polyéthylèneglycol est un polyéthylèneglycol ou un polyéthylèneglycolmonoalkyléther présentant une masse molaire Mn d'au moins 200 g/mole.

3. Composition selon la revendication 1 ou 2, **caractérisée en ce que** le monomère polaire, éthyléniquement insaturé est la vinylpyrrolidone, l'acide (méth)acrylique, un monomère contenant de l'acide sulfonique, un monomère aminofonctionnel ou un ester de l'acide (méth)acrylique d'un dérivé de polyéthylèneglycol.

4. Composition selon l'une quelconque des revendications 1 à 3, **caractérisée en ce que** le polymère en forme de peigne contient un mono(méth)acrylate de polyéthylèneglycol ou un alcoxylate d'alcool allylique sous forme polymérisée.

5. Composition selon l'une quelconque des revendications 1 à 4, **caractérisée en ce que** l'unité fonctionnelle en C₁-C₂₄ est liée par covalence au moyen du lieur et est choisie parmi les acides hydroxycarboxyliques, les acides aminocarboxyliques, les acides hydroxysulfoniques, les hydroxysulfates, les acides aminosulfoniques ou les aminosulfates, les hydroxyamines (telles que la diéthanolamine), les polyamines (par exemple la diéthylènetétraamine) ou les polyols (par exemple le glycérol, le triméthylolpropane, le pentaérythritol).

6. Composition selon l'une quelconque des revendications 1 à 5, **caractérisée en ce que** l'amphiphile contient un polycarbonate hyperramifié, qui est lié à au moins un polymère linéaire ou en forme de peigne.

7. Composition selon l'une quelconque des revendications 1 à 6, **caractérisée en ce que** la substance active contient une substance active agrochimique pharmaceutique.

8. Amphiphile contenant contient un polycarbonate hyperramifié, qui est lié à au moins un polymère linéaire ou en forme de peigne, **caractérisé en ce que** le polymère linéaire est
a) un homopolymère ou un copolymère statistique contenant un monomère polaire, éthyléniquement insaturé,
b) un polymère à blocs contenant un bloc de polyéthylèneglycol ou à base d'un monomère polaire, éthyléniquement insaturé,
c) un polycondensat ou un produit de polyaddition contenant du polyéthylèneglycol ou
d) un polyéthylèneglycol,
le polyéthylèneglycol d) étant lié via un lieur au polycarbonate,
le lieur étant un polyisocyanate présentant une fonctionnalité, en ce qui concerne les groupes isocyanate, d'au moins 1,5 et choisi parmi les diisocyanates et les polyisocyanates aliphatiques, cycloaliphatiques et aromatiques ainsi que les isocyanurates, les allophanates, les uretdiones et les biurets de diisocyanates aliphatiques, cycloaliphatiques et aromatiques, et
le polycarbonate contenant un alcool (B1), qui est un polyétherol trifonctionnel ou de fonctionnalité supérieure à base d'alcools, qui présentent au moins trois groupes OH, et d'oxyde d'alkylène en C₃-C₂₄.

9. Amphiphiles selon la revendication 8, **caractérisés en ce que** le polycarbonate peut être obtenu par
a) préparation d'un produit de condensation (K) par transformation d'un carbonate organique (A) ou d'un dérivé de phosgène avec l'alcool (B1), qui présente au moins trois groupes hydroxy, et
b) transformation intermoléculaire de K en polycarbonate hyperramifié,
le rapport des quantités des groupes OH aux groupes phosgène ou carbonate étant choisi de manière telle que K présente, en moyenne, soit i) un groupe carbonate ou un groupe chlorure de carbamoyle et plus d'un groupe OH, soit ii) un groupe OH et plus d'un groupe carbonate ou carbamoyle.

10. Amphiphile selon la revendication 8 ou 9, **caractérisé en ce que** le polymère en forme de peigne contient un mono(méth)acrylate de polyéthylèneglycol sous forme polymérisée.

11. Amphiphile selon l'une quelconque des revendications 8 à 10, **caractérisé en ce que** le polyéthylèneglycol est un polyéthylèneglycol ou un polyéthylèneglycolmonoalkyléther présentant une masse molaire Mn d'au moins 200 g/mole.

12. Procédé pour la préparation de l'amphiphile selon l'une quelconque des revendications 8 à 11, en ce qu'on transforme le polycarbonate, le polymère linéaire ou en forme de peigne et un lieur.

13. Utilisation de l'amphiphile selon l'une quelconque des revendications 8 à 11 dans des compositions contenant une substance active, qui est soluble dans l'eau à 20°C à raison d'au plus 10 g/l, pour la solubilisation de la substance active en milieu aqueux.

14. Utilisation de l'amphiphile selon l'une quelconque des revendications 8 à 11 pour lutter contre les champignons phytopathogènes et/ou une croissance non souhaitée de plantes et/ou une attaque non souhaitée par des insectes ou des acariens et/ou pour la régulation de la croissance de plantes, où on laisse agir l'amphiphile sur les différents nuisibles, leur espace de vie ou les plantes, le sol à protéger contre les différents nuisibles et/ou sur les plantes non souhaitées et/ou sur les plantes utiles et/ou leur espace de vie.

15. Matière végétale de multiplication contenant l'amphiphile selon l'une quelconque des revendications 8 à 11.
